Europäisches Patentamt

European Patent Office

Office européen des brevets

(19)

(11) Veröffentlichungsnummer: **0 285 675**
**A1**

# (12) EUROPÄISCHE PATENTANMELDUNG

(21) Anmeldenummer: 87101633.3

(22) Anmeldetag: 06.02.87

(51) Int. Cl.4 **C12N 15/00 , C07K 13/00 ,**
**C12P 21/02 , C12P 19/34**

Ein Antrag gemäss Regel 88 EPÜ auf Berichtigung der Seiten 20 und 23 der Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

Die Bezeichnung der Erfindung wurde geändert (Richtlinien für die Prüfung im EPA, A-III, 7.3).

(43) Veröffentlichungstag der Anmeldung:
**12.10.88 Patentblatt 88/41**

(84) Benannte Vertragsstaaten:
**AT BE CH DE ES FR GB GR IT LI LU NL SE**

(71) Anmelder: **BIOSYNTECH Biochemische**
**Synthesetechnik GmbH**
**Stresemannstrasse 268-280**
**D-2000 Hamburg 50(DE)**

(72) Erfinder: **Köster, Hubert, Dr.**
**1640 Monument Street**
**Concord MA 01742(US)**
Erfinder: **Biernat, Jacek, Dr.**
**Zeughausstrasse 12**
**D-2000 Hamburg 11(DE)**
Erfinder: **Kennedy. Nick, Dr.**
**Seidenweg 54**
**D-4000 Düsseldorf 13(DE)**
Erfinder: **Hofer,Bernd, Dr.**
**Leibnizstrasse 8**
**D-3340 Wolfenbüttel(DE)**

(74) Vertreter: **Henkel, Feiler, Hänzel & Partner**
**Möhlstrasse 37**
**D-8000 München 80(DE)**

(54) **Synthetische DNA-Kassetten mit Kodierung fuer artifizielle Proteine und deren Expression.**

(57) Beschrieben wird die Herstellung vollsynthetischer Proteine, deren Aminosäuresequenz unter Beachtung medizinischer oder ernährungsphysiologischer Gesichtspunkte genau dem jeweiligen Einsatzgebiet angepaßt ist, auf gentechnologischem Wege. Ausgegangen wird hierbei von das herzustellende Protein vollständig oder teilweise codierenden DNA-Sequenzen (sog. Kassetten), die alleine oder in beliebiger Kombination ektoren, z. B. Plasmide, integriert und in dieser Form in geeignete Wirte, z. B. Prokaryonten oder Euka nten einschließlich Pflanzen, transformiert werden. Die erhaltenen Wirt-Vektor-Systeme exprimieren das jeweilige Protein. Die Kassetten können auch mutagenisiert werden, d. h. bestimmte Codewörter nicht enthalten, oder oligomerisiert sein. Die Oligomerisierung ist beschrieben.

EP 0 285 675 A1

# Artifizielle Proteine definierter Aminosäurezusammensetzung, Verfahren zu ihrer Herstellung durch synthetische Nucleinsäuren sowie dabei verwendete DNA-Kassetten, Expressionsvektoren und Wirt-Vektor-Systeme

Eiweißstoffe sind neben Fetten und Kohlenhydraten wichtige Bestandteile von Lebensmitteln. Nach enzymatischer Hydrolyse zu den Aminosäuren im Verdauungstrakt liefern sie die für die Proteinbiosynthese notwendigen Bausteine. Nach ernährungsphysiologischen Gesichtspunkten werden sie in die - essentiellen Aminosäuren:

Valin (Val, V)

Leucin (Leu, L)

Isoleucin (Ile, I)

Phenylalanin (Phe, F)

Tryptophan (Trp, W)

Methionin (Met, M)

Threonin (Thr, T)

Lysin (Lys, K)

- quasiessentiellen Aminosäuren

Histidin (His, H)

Arginin (Arg, R) und

- nichtessentiellen Aminosäuren

Glycin (Gly, G)

Alanin (Ala, A)

Prolin (Pro, P)

Serin (Ser, S)

Cystein (Cys, C)

Tyrosin (Tyr, Y)

Asparagin (Asn, N)

Glutamin (Gln, Q)

Asparaginsäure (Asp, D)

Glutaminsäure (Glu, E)

eingeteilt. In Klammern sind die im Folgenden verwendeten üblichen Dreibuchstaben-und Einbuchstaben-Abkürzungen angegeben.

Die biologische Wertigkeit eines Proteins (g gebildetes Körperprotein / 100 g Nahrungsprotein) wird durch den absoluten Gehalt an essentiellen Aminosäuren bestimmt, aber auch durch das Mengenverhältnis der essentiellen Aminosäuren zueinander und zu den nicht-essentiellen Aminosäuren (H.-D. Belitz, W. Grosch, Lehrbuch der Lebensmittelchemie, Springer Verlag, 1982). Die biologische Wertigkeit wird im allgemeinen limitiert durch

- Lysin : Defizit bei Getreideproteinen und anderen pflanzlichen Proteinen,
- Methionin : Defizit bei Kuhmilch-und Fleischproteinen.
- Threonin : Defizit bei Weizen und Roggen.
- Tryptophan: Defizit bei Casein, Mais, Reis.

Es gibt kein natürliches Protein, das im Hinblick auf die biologische Wertigkeit optimal ist. Ein Gemisch aus 35 % Eiprotein und 65 % Kartoffelprotein nähert sich dem noch am weitesten an ("Kartoffel-Ei-Muster", E. Kofranyi und E. Jekat (1964) in Hoppe-Seyler's Z. physiol. Chem. 335, 174). Von der FAL/WHO wurde die optimale Zusammensetzung eines Referenzproteins definiert (FAO-Nutrition Meetings Report Series No. 37, WHO Technical Report Series No. 301, Rome, 1965). Ein Protein mit hoher biologischer Wertigkeit, bei dem also die Aminosäuren in den Mengen dem Organismus zugeführt werden, wie er sie für den Aufbau körpereigener Proteine benötigt, sollte demzufolge nach pharmako-kinetischen Gesichtspunkten konzipiert sein, so daß keine Imbalanzen (Überschüsse einiger Aminosäuren, die über eine Belastung des Organismus entgiftet werden müssen oder Defizite, die zur Verminderung der Proteinbiosynthese körpereigener Proteine führen müssen) auftreten können (F. Eckart, P.-U. Heuckenkamp und B. Weinheimer, Grundlagen und neue Aspekte der parenteralen und Sondenernährung, in der Reihe INA des G. Thieme Verlages, 1978, S. 243, 259 ff.). Derartige Proteine wären daher von großem physiologischen Wert, insbesondere für Krankenhausdiäten (A. Welsch, Krankenernährung, Thiemeverlag, 1982). Ein Protein, das allein die essentiellen und quasi-essentiellen Aminosäuren enthält, hätte große Bedeutung für die Verbesserung der biologischen Wertigkeit natürlicher Proteine für die Ernährung von Tieren und Menschen.

Für bestimmte Krankheitsbilder wie z.B. der Leberinsuffizienz (z.B. bei Hepatitis, Leberzirrhose) ist der Aminosäurestoffwechsel gestört. Dies führt dazu, daß im Blutplasma die aromatischen Aminosäuren Phe, Tyr und Trp, sowie Met gegenüber normalen Verhältnissen erhöht, während die verzweigtkettigen Aminosäuren Leu, Val, Ile erniedrigt sind (F. Ch. Bode (1978), Internist 19, 72; E. Holm, Behandlungen mit Aminosäuren bei hepatischer Enzephalopathie, Fischerverlag, 1976; J. Eckart, P.-U. Heuckenkamp und B. Weinheimer (1978), Thiemeverlag, S. 285 ff). Hier ist die richtige Aminosäurezusammensetzung besonders wichtig. Eine unter normalen Bedingungen optimale Aminosäurezusammensetzung führt hier zu einer Verschlechterung des Krankheitsbildes unter Zunahme hepatischer Enzephalopathien. Mit einer bedarfsadaptierten Aminosäurezusammensetzung hingegen kann Leberinsuffizienz wirksam therapiert werden. Deratige Proteine mit einer in dieser speziellen Hinsicht optimalen Aminosäurezusammensetzung kommen in der Natur nicht vor.

Ein anderer Fall, bei dem das Krankheitsbild entscheidend von der Aminosäurezusammensetzung der in den Lebensmitteln vorhandenen Eiweißstoffe beeinflußt wird, ist eine Form des Schwachsinns, der die Folge eines vererbten Stoffwechseldefektes ist: Phenylketonurie (G.G. Wendt, Vererbung und Erbkrankheiten (1974), Herder & Herder; H. Stengel: Humangenetik (1976), Quelle & Meyer). Durch eine Phenylalaninfreie Diät, die allerdings von der Geburt an verabreicht werden muß, kann diese Erbkrankheit individuell geheilt werden. Ein Protein, das alle notwendigen Aminosäuren enthält, aber frei von Phenylalanin ist, ist in der Natur unbekannt.

Um Aminosäurezusammensetzungen entsprechend dem FAO /WHO-Referenzprotein oder dem Kartoffel-Ei-Muster sowie zur Behandlung der Leberinsuffizienz und Phenylketonurie bereitzustellen sind bisher folgende Wege beschritten worden:

1) Verwendung von Aminosäuregemischen:

sie stehen für alle drei erwähnten Einsatzgebiete zur Verfügung. Von großem Nachteil sind:
- die sehr unterschiedliche Löslichkeit einzelner Aminosäuren , wobei insbesondere die geringe Löslichkeit von Cystein, Tryptophan, Tyrosin, Asparagin-und Glutaminsäure, Leucin und Phenylalanin Probleme bereitet.
- der bittere Geschmack
- Empfindlichkeit beim Erhitzen (z.B. Sterilisieren). Eine Zersetzung führt z.T. zu toxischen Substanzen und zur Herabsetzung der biologischen Wertigkeit.
- führt bei oraler Applikation zu Problemen
- hohe Osmolarität setzt die dem Organismus zumutbaren Mengen stark herab.

Die meisten der beschriebenen Nachteile wären nicht mehr existent, wenn die jeweils optimalen Aminosäurezusammensetzung in Form von Peptiden oder Proteinen vorlägen. Hinzukommt das Peptide besser vom Darmtrakt resorbiert werden als die freien Aminosäuren (S.A. Adibi und Y.S. Kim, Peptide Absorption and Hydrolysis (1981) Physiology of the Gastrointestinal Tract, edited by Leonard R. Johnson, Raven Press).

2) Chemische Peptidsynthese:

Dieses Verfahren ist zu aufwendig und daher wirtschaftlich nicht zu vertreten.

3) Enzymatische Veränderung von natürlichen Proteinen.

Hier ist insbesondere die Plasteinreaktion zu nennen, die durch das Zusammenwirken von enzymatischen Hydrolysen und enzymatischen Peptidsynthesen das Verhältnis der natü        Aminosäurezusammensetzung eines Proteins zu verändern gestattet (H.D. Belitz, W. Grosch (19   Jahrbuch der Lebensmittelchemie, S. 65 ff). Es wurde sowohl der Anteil einiger essentieller Aminosäure: (K. Aso et al. (1974) Tryptophan-, threonine-und lysine-enriched plasteins from Zein, Agric. Biol.Chem. 38, 679) erhöht als auch der Anteil von Phenylalanin für Phenylketonurie-Patienten erniedrigt (M. Yamashita et al. (1976), A low-phenyl-alanine, high-tyrosine plastein as an acceptable dietic food. J. Food Science 41, 1029). Nachteil dieses Verfahrens ist der immer noch recht hohe Aufwand und die Tatsache, daß undefinierte und schwer reproduzierbare Produkte entstehen.

Der Erfindung liegt nun die Aufgabe zugrunde, auf einfache Weise molekular einheitliche Proteine mit genau für das entsprechende Einsatzgebiet definierter Aminosäurezusammensetzung in hoher Reproduzierbarkeit bereitzustellen.

Die Lösung dieser Aufgabe beruht auf dem Einsatz gentechnischer Methoden.

Durch Gensynthese kann über den genetischen Code ein Protein-Gen synthetisiert werden, das über die definierte, codierte Aminosäuresequenz nach üblichen gentechnischen Methoden das gewünschte Protein liefert.

Dies gelang z.B. für das Peptid Somatostatin (K. Itakura et al. (1977), Science 198, 1056) und für ein artifizielles Gen, das für repititive Einheiten der beiden Aminosäuren (Asp-Phe)$_n$ codiert (M.T. Doel et al. (1980), Nucleic Acids Res. 8, 4575 und DE-A-3102721). Das zuletzt genannte Verfahren liefert ein Gemisch von Polypeptiden, bei denen n bis zu 150 betragen kann. Es konnte nach Polymerisation von zwei Dodekanucleotiden, anschließender Integration des Gemisches der Oligomeren in ein Plasmid und Transformation von E. coli-Zellen nach Expression gewonnen werden. S.C. Gupta et al. (1983) berichten in Biotechnology, auf Seite 602 ff. über den Versuch durch ein selbstkomplementäres Dodekanucleotid auf dem prinzipiell gleichen Weg ein Polypeptid herzustellen, das aus repetitiven Einheiten der Aminosäuren (Phe-Trp-Pro-Lys) besteht. Auch hier würde das Verfahren in der Länge uneinheitliche Produkte liefern. Ihnen gelang die Polymerisation des 12-mers zu einem Doppelstrang von ca. 1000 Basenpaaren. Nach Integration in ein Plasmid konnten nach Klonierung, jedoch - vermutlich aufgrund der unnatürlich hohen Repetition von Palindromsequenzen - für das Tetrapeptid codierende DNA-Sequenzen nur bis zu 120 Basenpaaren gefunden werden. Eine Expression, d.h. ein Nachweis, daß das Tetrapeptid oder ein Oligomeres davon tatsächlich von der E. coli-Zelle hergestellt wird, gelang ihnen nicht.

Aufgrund des heutigen Kenntnisstandes ist auch der Expressionserfolg von Genen, die für artifzielle, d.h. nicht durch die Evolution optimierte Proteine codieren, nicht planbar (F. van Brunt (1986), Biotechnology, 277).

Durch die im folgenden näher erläuterten erfindungsgemäßen Verfahren erhält man hingegen erstmalig definierte Proteine, die z.B.

1) nur für die essentiellen Aminosäuren (p-A = Protein A),

2) für ein phenylalanin-freies Protein mit ansonsten optimaler Aminosäurezusammensetzung (p-AF⁻B) für die Behandlung der Phenylketonurie und

3) für ein Protein mit optimaler Aminosäurezusammensetzung zur Behandlung der Leberinsuffizienz (p-AHB mit einem hohen Anteil an verzweigtkettigen Aminosäuren codieren.

Derartige Proteine sind weder in der Natur bekannt noch bisher auf irgendeinem anderen möglichen Wege in wirtschaftlich vertretbarer Form herstellbar gewesen. Sie stellen daher völlig neue Proteine dar.

Die erfindungsgemäßen artifiziellen Proteine mit unterschiedlichen Anwendungsgebieten werden durch gentechnische Methoden gewonnen. Die entsprechend codierten Aminosäuresequenzen wurden mit dem Genetischen Code in DNA-Sequenzen übersetzt (B. Alberts et al., 1983, Molecular Biology of the Cell, Garland Publishing, Inc.). Um Flexibilität auf dem DNA-Klonierungsniveau zu gewährleisten und um nach dem Baukastenprinzip verschiedene Proteine produzieren zu können, wurde eine Strategie gewählt, bei der austauschbare DNA-Kassetten für bestimmte Gruppen von Aminosäuren codierten.

Das Konzept von DNA-Kassetten, die nur für Proteine kodieren, die aus einer bestimmten Gruppe von Aminosäuren bestehen, erlaubt über die Kassettenmutagenese und Oligomerisierung (s. weiter unten) einzelner Kassetten eine Vielzahl von Kombinationsmöglichkeiten.

Die Erfindung wird im folgenden anhand verschiedener Kassettentypen oder Kombinationen hiervon näher erläutert.

Kassette A codiert nur für die essentiellen, B nur für die nicht-essentiellen und H für die verzweigtkettigen Aminosäuren. Desweiteren wurde z.B. eine Kassette AF⁻ konzipiert, die alle essentiellen Aminosäuren in gleicher molprozentualen Verteilung enthielten mit Ausnahme von Phenylalanin. Durch Kombination dieser Kassetten waren die Strukturgene mit Codierung für folgende Proteine zugänglich:

1) Ein Protein, das nur aus essentiellen Aminosäuren besteht (p-A),

2) ein Protein (p-AB), das alle Aminosäuren in ernährungsphysiologisch optimaler Verteilung enthält,

3) ein Protein (p-AF⁻B), das für die Behandlung von Phenylketonurie (PKU) geeignet ist, da mit Ausnahme von Phenylalanin, alle Aminosäuren in physiologisch optimaler Verteilung vorliegen und

4) ein Protein (p-AHB) mit einem hohen Anteil an den drei verzweigtkettigen Aminosäuren Val, Leu, Ile zur Behandlung von Leberinsuffizienzen. Die Zusammensetzung der vier Proteine zeigt Tabelle 1.

Die Aminosäure Tyrosin zählt nicht im engeren Sinne zu den für den Menschen essentiellen Aminosäuren. Sie wurde aus praktischen Gründen in das 5′-Ende der A-Kassette integriert. Die gezielte Entfernung des Codewortes für Tyr gelingt leicht auf dem Wege der Kassettenmutagenen, wie später am Beispiel der AF⁻-Kassette gezeigt werden wird.

## Tabelle 1

Aminosäurezusammensetzung der artifiziellen Proteine p-A, p-AB, p-AF⁻B und p-AHB, angegeben als Aminosäurereste pro Molekül.

| | p-A | p-AB | p-AF⁻B | p-AHB |
|---|---|---|---|---|
| Isoleucin | 3 | 3 | 3 | 7 |
| Leucin | 4 | 4 | 4 | 9 |
| Valin | 3 | 3 | 3 | 9 |
| Lysin | 2 | 2 | 2 | 2 |
| Methionin | 1 | 1 | 1 | 1 |
| Phenylalanin | 1 | 1 | - | 1 |
| Threonin | 3 | 3 | 3 | 3 |
| Tryptophan | 1 | 1 | 1 | 1 |
| Tyrosin | 1 | 1 | 1 | 1 |
| Arginin | 2 | 2 | 2 | 2 |
| Histidin | 2 | 2 | 2 | 2 |
| Alanin | - | 10 | 10 | 10 |
| Asparaginsäure | - | 2 | 2 | 2 |
| Cystein | - | 1 | 1 | 1 |
| Glutaminsäure | - | 2 | 2 | 2 |
| Glycin | - | 4 | 4 | 4 |
| Prolin | - | 3 | 3 | 3 |
| Serin | - | 2 | 2 | 2 |

Für die Bestimmung der ernährungsphysiologisch optimalen Aminosäurezusammensetzungen wurde das FAO/WHO Referenzprotein, das Kartoffel-Ei-Muster und verschiedene kommerziell erhältliche Aminosäurelösungen (z.B. Albumacid XP, Aponti PKU, Milupa PKU, Falkamin und Braun APL) zugrundegelegt. Asparagin und Glutamin wurden nicht berücksichtigt, da sie nicht essentiell sind, aus Asp bzw. Glu vom Organismus hergestellt werden können und ein zu großer Anteil unter Umständen metabolische Probleme mit sich bringen kann.

Die einzelnen DNA Kassetten wurden nach dem "filling-in"-Konzept (F.F. Rossi et al. 1982, J. Biol. Chem. 257, 9226) mit dem Klenow-Enzym und unter Zugrundelegung einer Überlappungslänge der einzelsträngigen DNA-Fragmente von 12 Basenpaaren hergestellt. Die einzelsträngigen DNA-Fragmente wurden nach der β-Cyanoethylphosphoramiditmethode (H. Köster et al. 1984, Nucleic Acids Res. 12, 4539) durch chemische Synthese dargestellt. Restriktionserkennungsstellen wurden derart positioniert, daß die einzelnen Kassetten in definierter Weise miteinander unter Beibehaltung des Leserasters des Genetischen Codes kombiniert werden können. Das Codewort für z.B. Phenylalanin wurde am 5'-Ende der A-Kassette lokalisiert, so daß damit die Synthese des Gens für p-A und p-AF⁻ miteinander kombiniert werden konnte. (Entsprechendes gilt für das Codewort für Trp, Tyr.)

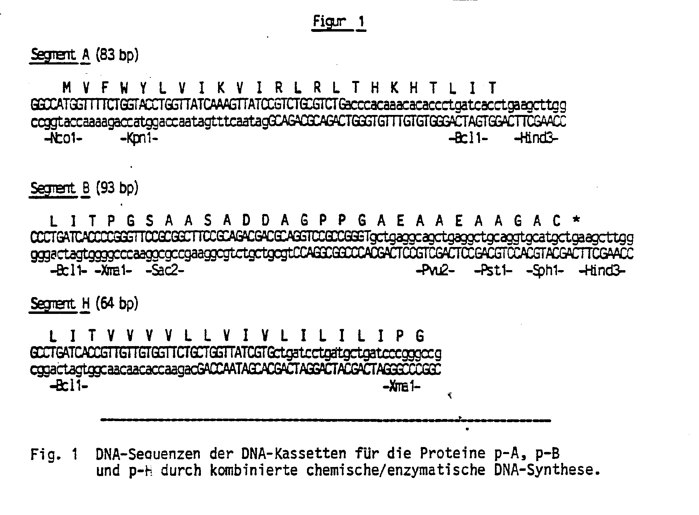

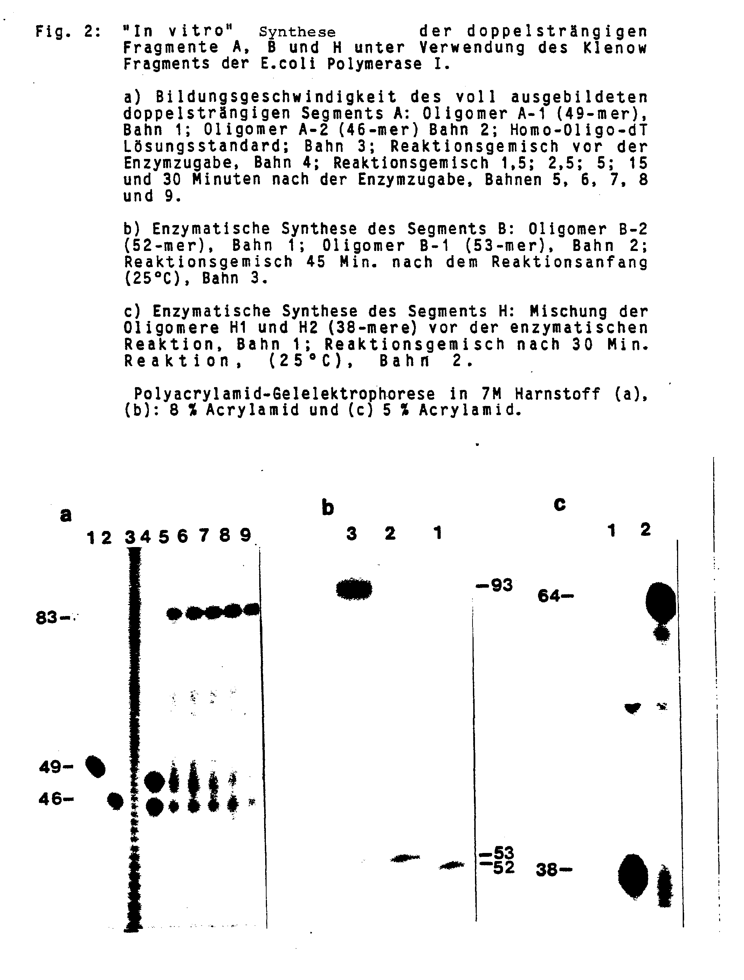

Die drei Segmente für die A, B und H-Kassetten zeigt Fig.1, wobei die großen Buchstaben die durch chemische, die kleinen Buchstaben die durch enzymatische Synthese eingebauten Bausteine (Nucleotide) repräsentieren. Über dem DNA-Doppelstrang (bp = Basenpaar) steht die codierte Aminosäuresequenz im Einbuchstaben-Code. Die entsprechenden Restriktionsendonuklease-Erkennungssequenzen sind in Bindestrichen mit den üblichen Abkürzungen vermerkt. Das Segment, das für p-AF⁻ codiert unterscheidet sich vom A-Segment durch das Fehlen von Phenylalanin (F) am 5′-Ende des Duplexes. Die enzymatische Synthese der A-, B-und H-Kassette zeigt Fig.2.

Im folgenden wird zunächst erläutert, wie die synthetischen DNA-Kassetten zur Gewinnung expressionsfähiger Vektoren gereinigt und vermehrt werden. Zu diesem Zweck wird die jeweilige Kassette oder Kassettenkombination in ein bekanntes Plasmid integriert und später "in gereinigter Form" zur Integration in eines der erfindungsgemäß benutzten Plasmide wieder ausgeschnitten.

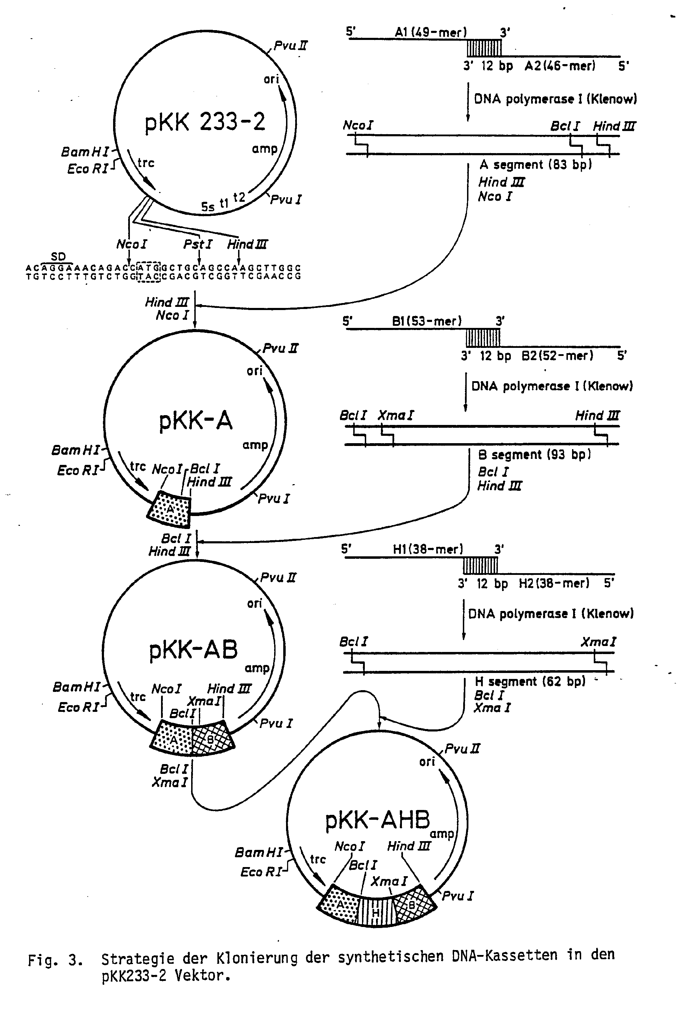

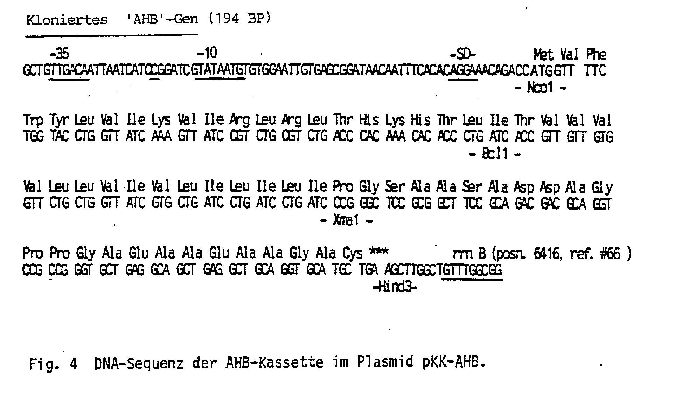

Die Klonierungsstrategie geht aus Fig. 3 hervor. Ein Doppelverdau des Plasmids pKK233-2 (E. Aman und J. Brosius (1985), Gene 40, 183) mit den Enzymen Ncol und HindIII erlaubte den Einbau der entsprechend enzymatisch behandelten Kassette A mit T4 Polynucleotidligase und führte zum Plasmid pKK-A (bzw. pKK-AF⁻, wenn die Kassette AF⁻ verwendet wurde). In der in Abb. 3 gezeigten Weise wurde nun schrittweise zunächst Kassette B eingebaut. Hierdurch werden die Strukturgene für die Proteine p-A, p-AB und pAF⁻B zugänglich. Einbau der Kassette H in das Plasmid PKK-AB lieferte das Strukturgen für das Protein p-AHB. Fig. 4 zeigt den Sequenzausschnitt des Plasmids pKK-AHB in der Integrationsregion des AHB-Strukturgens. Die -35 und -10 Region des tac-Promoters und die Shine-Dalgarno-Sequenz (SD) -Ribosomenbindungsstelle -sowie Teile des rrnB-Gens, sind verzeichnet. Die verwendeten Plasmide pKK-A, pKK-AB, pKK-AF⁻B und pKK-AHB wurden zum Klonieren der synthetischen Kassetten in E.coli GM33 (dam⁻) bzw., HB101 verwendet. Die korrekte Integration sowie die Richtigkeit der DNA-Sequenz wurden durch Sequenzierung nach Maxam und Gilbert bestimme (A.M. Maxam und W. Gilbert (1980), Methods in Enzym. 65, 444).

Das Kassetten-Konzept erlaubt nicht nur die in Fig. 3 dargestellte Vorgehensweise, sondern ermöglicht auch mit kleinen Ergänzungen durch geeignete Linker-Sequenzen die Kassetten einzeln zu oligomerisieren und gegebenfalls nun zu kombinieren. Dies führt zur Erhöhung des Molekulargewichts des gewünschten Produktes und über einen "gene dosage effect" auch zur Erhöhung der Menge an hergestelltem Produkt pro Bakterienzelle. Hinzu kommt, daß kurze, fremde Proteine nicht selten in Mikroorganismen instabil sind. Darüberhinaus eröffnet die Kombination einzelner oligomerisierter Kassetten mit anderen Kassetten (vor und nach Oligomerisierung) die Einstellung anderer Aminosäurezusammensetzungen des codierten Proteins. Im folgenden sind einige Beispiele von Kassettenoligomerisierung und -mutagenese (s. weiter unten) gegeben:

$p-A_2B$, $p-A_3B$, $p-A_6B$, $p-A_{12}B$

$p-AB_2$, $p-AB_3$, $pAB_6$, $p-AB_{12}$

$p-A_2HB$, $p-A_6HB$, $p-AHB_2$, $p-AHB_6$,

$p-AF⁻HB$, $p-AW⁻HB$, $p-AHB/AW⁻HB$

$p-AW⁻HB/AHBC⁻$

$p-AH_2B$, $p-A_2H_2B$, $p-AH_2B_2$

und zur Vergrößerung des Molekulargewichtes mit einhergehender Stabilisierung der Proteinstruktur:

$p-A_2B_2$, $p-A_3B_3$, $p-A_6B_6$, $p-A_{12}B_{12}$

$p-A_2H_2B_2$, $p-A_3H_3B_3$, $p-A_6H_6B_6$

Allgemein geschieht die Oligomerisierung von DNA-Sequenzen schrittweise, indem man

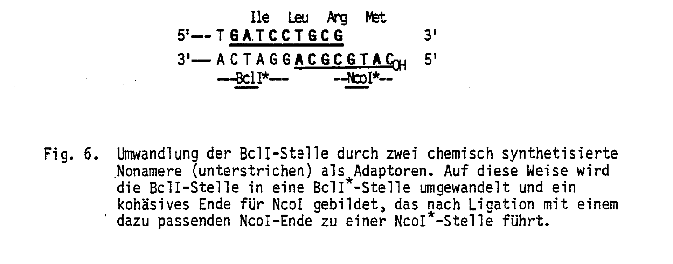

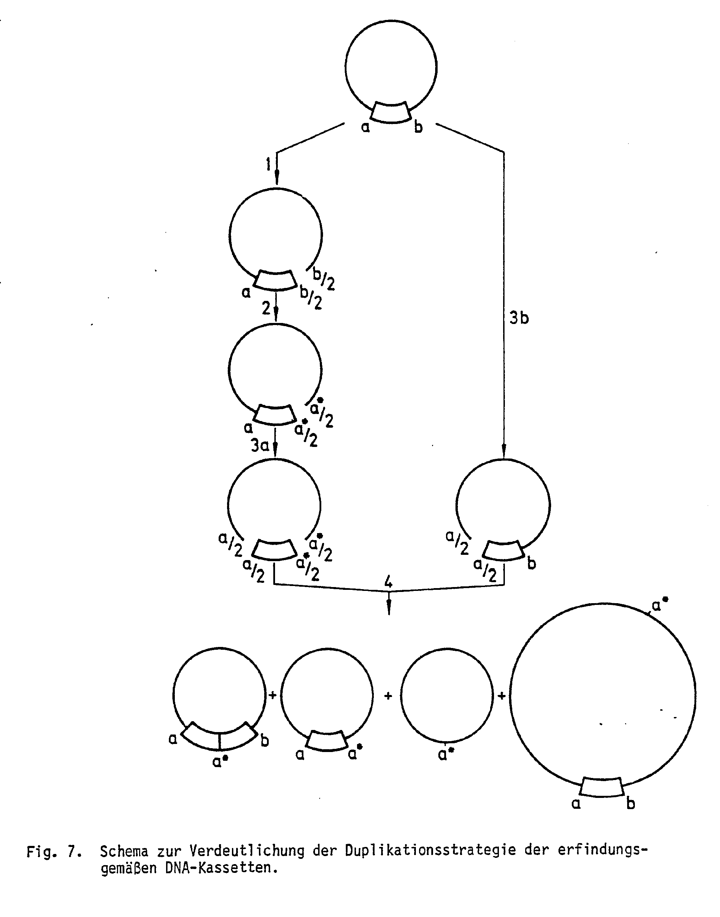

A) die in ein Plasmid integrierte und zu oligomerisierende Fremd-DNA auf einer Seite am 3′ -oder 5′ -Ende (a oder b) schneidet (Stufe 1 von Fig. 7); an beide Enden der geöffneten Fremd-DNA Adaptermoleküle anligiert (Stufe 2 von Fig. 7), wobei die Adaptermoleküle die Spezifität der kohäsiven Enden der nicht geöffneten Seite der Fremd-DNA ohne Möglichkeit zur Restaurierung der Restriktionserkennungsstelle herstellen (Fig. 6), die Fremd-DNA am (anderen) 5′-oder 3′-Ende (b oder a) schneidet (Stufe 3a in Fig. 7), und die noch vorhandenen 5′-OH-Enden mit T4-Polynucleotidkinase phosphoryliert;

B) ein Aliquot zu der in das Plasmid integrierten und zu oligomerisierenden Fremd-DNA auf der anderen Seite schneidet (Stufe 3b von Fig. 7), und durch Behandeln mit Alkalischer Phosphatase Dephosphoryliert;

C) die beiden Ansätze aus den Verfahrensstufen A) und B) miteinander vereinigt und mit Hilfe von DNA-Ligasen ligiert (Stufe 4 von Fig. 7);

D) eine Klonierung durchführt;

E) den Klon mit der dublizierten Fremd-DNA isoliert und

F) gegebenenfalls die Verfahrensstufen A) bis C) beliebig oftwiederholt oder

nach Durchführung der Verfahrensstufe A)

B) die vom Plasmid befreite bzw. isolierte Fremd-DNA in Gegenwart der entsprechenden Restriktionsenzyme (mit entweder a-oder b-Spezifität) durch T4-Polynucleotidligase ligiert und gegebenenfalls die Schritte A) und B) wiederholt;

C) die so erhaltenen Oligomere in ein geeignetes Plasmid integriert und nach Transformation in geeignete E. coli Stämme die oligomerisierte Fremd-DNA kloniert.

Detaillierte Beispiele für den Einsatz mutagenisierter und/oder oligomerisierter Kassetten vermitteln Tabellen

Tabelle 2

| | Reste | A | AF⁻ | AW⁻ | AY⁻ | AM⁻ | AB Mol - % | AF⁻B | ABC⁻ | AW⁻B | AY⁻B | AM⁻B | Reste | AHB | AW⁻HB Mol - % | AHBC⁻ |
|---|---|---|---|---|---|---|---|---|---|---|---|---|---|---|---|---|
| Ile | 3 | 13.0 | 13.6 | 13.6 | 13.6 | 13.6 | 6.4 | 6.5 | 6.5 | 6.5 | 6.5 | 6.5 | 7 | 11.3 | 11.5 | 11.5 |
| Leu | 4 | 17.4 | 18.2 | 18.2 | 18.2 | 18.2 | 8.5 | 8.7 | 8.7 | 8.7 | 8.7 | 8.7 | 9 | 14.5 | 14.8 | 14.8 |
| Val | 3 | 13.0 | 13.6 | 13.6 | 13.6 | 13.6 | 6.4 | 6.5 | 6.5 | 6.5 | 6.5 | 6.5 | 9 | 14.5 | 14.8 | 14.8 |
| Lys | 2 | 8.7 | 9.1 | 9.1 | 9.1 | 9.1 | 4.3 | 4.3 | 4.3 | 4.3 | 4.3 | 4.3 | 2 | 3.2 | 3.3 | 3.3 |
| Met | 1 | 4.3 | 4.5 | 4.5 | 4.5 | - | 2.1 | 2.2 | 2.2 | 2.2 | 2.2 | - | 1 | 1.6 | 1.6 | 1.6 |
| Phe | 1 | 4.3 | - | 4.5 | 4.5 | 4.5 | 2.1 | - | 2.2 | 2.2 | 2.2 | 2.2 | 1 | 1.6 | 1.6 | 1.6 |
| Thr | 3 | 13.0 | 13.6 | 13.6 | 13.6 | 13.6 | 6.4 | 6.5 | 6.5 | 6.5 | 6.5 | 6.5 | 3 | 4.8 | 4.9 | 4.9 |
| Trp | 1 | 4.3 | 4.5 | - | 4.5 | 4.5 | 2.1 | 2.2 | 2.2 | - | 2.2 | 2.2 | 1 | 1.6 | - | 1.6 |
| Tyr | 1 | 4.3 | 4.5 | 4.5 | - | 4.5 | 2.1 | 2.2 | 2.2 | 2.2 | - | 2,2 | 1 | 1.6 | 1.6 | 1.6 |
| Arg | 2 | 8.7 | 9.1 | 9.1 | 9.1 | 9.1 | 4.3 | 4.3 | 4.3 | 4.3 | 4.3 | 4.3 | 2 | 3.2 | 3.3 | 3.3 |
| His | 2 | 8.7 | 9.1 | 9.1 | 9.1 | 9.1 | 4.3 | 4.3 | 4.3 | 4.3 | 4.3 | 4.3 | 2 | 3.2 | 3.3 | 3.3 |
| | 23 | 99.7 | 99.8 | 99.8 | 99.8 | 99.8 | 49.0 | 47.7 | | | | | | | | |
| Ala | 10 | | | | | | 21.3 | 21.7 | 21.7 | 21.7 | 21.7 | 21.7 | 10 | 16.1 | 16.4 | 16.4 |
| Asp | 2 | | | | | | 4.3 | 4.3 | 4.3 | 4.3 | 4.3 | 4.3 | 2 | 3.2 | 3.3 | 3.3 |
| Cys | 1 | | | | | | 2.1 | 2.2 | - | 2.2 | 2.2 | 2.2 | 1 | 1.6 | 1.6 | - |
| Glu | 2 | | | | | | 4.3 | 4.3 | 4.3 | 4.3 | 4.3 | 4.3 | 2 | 3.2 | 3.3 | 3.3 |
| Gly | 4 | | | | | | 8.5 | 8.7 | 8.7 | 8.7 | 8.7 | 8.7 | 4 | 6.5 | 6.6 | 6.6 |
| Pro | 3 | | | | | | 6.4 | 6.5 | 6.5 | 6.5 | 6.5 | 6.5 | 3 | 4.8 | 4.9 | 4.9 |
| Ser | 2 | | | | | | 4.3 | 4.3 | 4.3 | 4.3 | 4.3 | 4.3 | 2 | 3.2 | 3.3 | 3.3 |
| | 47 | | | | | | 100.2 | 99.7 | 99.7 | | | | | | | |
| Beispiel | | 1 | 2 | 3 | 4 | 5 | 6 | 7 | 8 | 9 | 10 | 11 | | 12 | 13 | 14 |

0 285 675

## Tabelle 3

| | A₂B | | A₆B | | A₁₂B | | AB₂ | | AB₆ | | AB₁₂ | | AHB₂ | | AHB/AW⁻HB | |
|---|---|---|---|---|---|---|---|---|---|---|---|---|---|---|---|---|
| | Reste | Mol% | Reste | Mol% | Reste | Mol% | Reste | Mol% | Reste | Mol% | Reste | Mol% | Reste | Mol% | Reste | Mol% |
| Ile | 6 | 8.6 | 18 | 11.1 | 36 | 12.0 | 3 | 4.2 | 3 | 1.8 | 3 | 1.0 | 7 | 8.2 | 14 | 11.4 |
| Leu | 8 | 11.4 | 24 | 14.8 | 48 | 16.0 | 4 | 5.6 | 4 | 2.4 | 4 | 1.3 | 9 | 10.5 | 18 | 14.7 |
| Val | 6 | 8.6 | 18 | 11.1 | 36 | 12.0 | 3 | 4.2 | 3 | 1.8 | 3 | 1.0 | 9 | 8.2 | 18 | 14.7 |
| Lys | 4 | 5.8 | 12 | 7.5 | 24 | 8.1 | 2 | 2.8 | 2 | 1.2 | 2 | 0.7 | 2 | 2.3 | 4 | 3.2 |
| Met | 2 | 2.8 | 6 | 3.7 | 12 | 3.9 | 1 | 1.4 | 1 | 0.6 | 1 | 0.3 | 1 | 1.2 | 2 | 1.6 |
| Phe | 2 | 2.8 | 6 | 3.7 | 12 | 3.9 | 1 | 1.4 | 1 | 0.6 | 1 | 0.3 | 1 | 1.2 | 2 | 1.6 |
| Thr | 6 | 8.6 | 18 | 11.1 | 36 | 12.0 | 3 | 4.2 | 3 | 1.8 | 3 | 1.0 | 3 | 3.5 | 6 | 4.9 |
| Trp | 2 | 2.8 | 6 | 3.7 | 12 | 3.9 | 1 | 1.4 | 1 | 0.6 | 1 | 0.3 | 1 | 1.2 | 1 | 0.8 |
| Tyr | 2 | 2.8 | 6 | 3.7 | 12 | 3.9 | 1 | 1.4 | 1 | 0.6 | 1 | 0.3 | 1 | 1.2 | 2 | 1.6 |
| Arg | 4 | 5.8 | 12 | 7.5 | 24 | 8.1 | 2 | 2.8 | 2 | 1.2 | 2 | 0.7 | 2 | 2.3 | 4 | 3.2 |
| His | 4 | 5.8 | 12 | 7.5 | 24 | 8.1 | 2 | 2.8 | 2 | 1.2 | 2 | 0.7 | 2 | 2.3 | 4 | 3.2 |
| Ala | 10 | 14.3 | 10 | 6.2 | 10 | 3.3 | 20 | 28.1 | 60 | 35.9 | 120 | 38.5 | 20 | 23.3 | 20 | 16.3 |
| Asp | 2 | 2.9 | 2 | 1.2 | 2 | 0.7 | 4 | 5.7 | 12 | 7.2 | 24 | 7.8 | 4 | 4.6 | 4 | 3.2 |
| Cys | 1 | 1.4 | 1 | 0.6 | 1 | 0.3 | 2 | 2.8 | 6 | 3.5 | 12 | 3.8 | 2 | 2.3 | 2 | 1.6 |
| Glu | 2 | 2.9 | 2 | 1.2 | 2 | 0.7 | 4 | 5.7 | 12 | 7.2 | 24 | 7.8 | 4 | 4.6 | 4 | 3.2 |
| Gly | 4 | 5.7 | 4 | 2.5 | 4 | 1.3 | 8 | 11.2 | 24 | 14.3 | 48 | 15.4 | 8 | 9.4 | 8 | 6.6 |
| Pro | 3 | 4.3 | 3 | 1.9 | 3 | 1.0 | 6 | 8.5 | 18 | 10.8 | 36 | 11.6 | 6 | 6.9 | 6 | 4.9 |
| Ser | 2 | 2.9 | 2 | 1.2 | 2 | 0.7 | 4 | 5.7 | 12 | 7.2 | 24 | 7.8 | 4 | 4.6 | 4 | 3.2 |
| | | 100.2 | | | | | | | | | | | | 21 | 123 | 99.9 |
| Beispiel | 15 | | 16 | | 17 | | 18 | | 19 | | 20 | | 21 | | 22 | |

Das Konzept ermöglicht natürlich noch eine Vielzahl anderer Kombinationen unter Einschluß auch der AF⁻, AW⁻-etc. -Kassette, die wegen ihrer Offensichtlichkeit nicht weiter ausgeführt zu werden brauchen.

Hierbei wird einschränkend zugrundegelegt, daß einzelne Kassetten oder Kassettenblöcke maximal 12-mal verknüpft werden sollen und einzelne Kassetten durch Kassettenmutagenese in der oben angegebenen Weise nicht mehr als maximal vier Codewörter verlieren sollen.

Stellvertretend für die oben erwähnten Möglichkeiten der Oligomerisierung wird die der A-Kassette im Detail beschrieben.

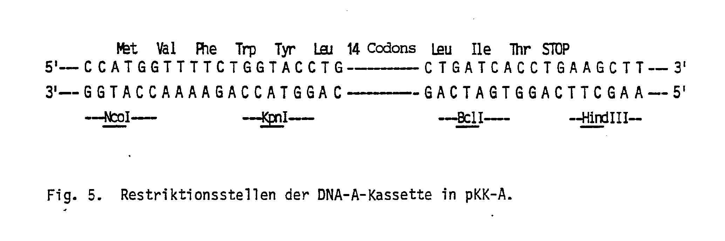

Die im Plasmid pKK-A integrierte DNA-Sequenz der A-Kassette zeigt Fig. 5.

Das Startcodon ATG liegt in der NcoI-Sequenz, während die BclI-Sequenz sich kurz vor dem Stopcodon TGA befindet. Eine Oligomerisierung soll daher zweckmäßigeweise zwischen diesen Positionen erfolgen, wobei natürlich das Leseraster der Proteinbiosynthese erhalten bleiben muß. Dies gelingt durch entsprechende Adapter-Moleküle, wie sie Fig. 6 zeigt. Das Plasmid pKK-A wurde mit BclI geöffnet und die BclI-Stelle durch zwei Adaptermoleküle (unterstrichen) in eine NcoI*-Stelle umgewandelt (NcoI*, da zwar komplementäre kohäsive Enden, entsprechend NcoI-Spezifität, jedoch würde Ligation nicht zur Rekonstitution einer NcoI-Stelle führen: CCATGC anstelle von CCATGG; entsprechend wurde die BclI-Stelle in bine BclI*-Stelle umgewandelt ). Dieses Konzept erlaubt zwei Möglichkeiten. Zum einen kann sas NcoI / NcoI* - Fragment in Gegenwart des Enzyms NcoI in gezielter Weise oligomerisiert werden. Zum anderen kann das gleiche Fragment für eine kontinuierliche Genduplikation verwendet werden (Fig. 7). Dies gelingt dadurch, daß das mit BclI linearisierte Plasmid pKK-A mit den beiden Adaptermolekülen ligiert wird. Dies wird verdeutlicht durch die Schritte 1, 2 und 3a von Fig. 7. Die BclI-Stelle wird dadurch in eine NcoI*-(und gleichzeitig auch die BclI in eine BclI*) Stelle umgewandelt. Ein anderer Teil des Plasmids pKK-A wird mit NcoI linearisiert (Schritt 3b in Fig. 7). Nun wird die NcoI-Stelle des ersten Plasmids (Schritt 3a) mit NcoI geschnitten und die Reaktionsprodukte nach Phosphorylierung der 5'-OH-Enden mit T4-Polynucleotidkinase mit dem dephosphorylierten Plasmid nach Schritt 3b (Fig. 7) vereinigt und einer Ligation mit T4-Polynucleotidligase unterworfen (Schritt 4 in Fig. 7). Die möglichen Reaktionsprodukte ergeben sich aus Figur 7. Das linke Plasmid zeigt die gewünschte Verdoppelung der A-Kassette und konnte durch Klonierung in E. coli GM 33 (dam⁻) erhalten werden.

Da das Dimere an der Verknüpfungsstelle der Monomeren weder eine BclI-noch NcoI-Sequenz besitzt (Fig. 6) kann es zur Gewinnung eines Trimeren, Hexameren etc. verwendet werden. Auf diesem Wege wird eine $A_6$-Kassette hergestellt:

$$A + A \rightarrow A_2 + A \rightarrow A_3 + A_3 \rightarrow A_6$$

Die jeweiligen Plasmid-Konstruktionen wurden in E.coli HB101 bzw. GM33 (erforderlich wenn mit BclI geschnitten werden soll, und dafür eine unmethylierte BclI-Stelle vorliegen muß) kloniert. Die jeweilige Oligomerisierung konnte durch Behandlung der Plasmid-DNA mit NcoI / HindIII und Vergleich der erhaltenen Bruchstücke mit Längenmarker nachgewiesen werden.

Die Expression der Proteine p-A, p-AB, p-AF⁻B, p-AHB wurde im Plasmid pGFY221N und pBI052 durchgeführt. pGFY221N wurde aus dem Vektor pGFY218L (K. Talmadge und W. Gilbert (1980), Gene 12, 235) durch folgende Operationen erhalten:

1) Verdauung mit PstI.
2) Entfernen der überstehenden 3'-Enden mit dem Klenow-Enzym.
3) Ligation des Linkers d(AGCCATGGCT) an die stumpfen Enden des Plasmids.
4) Verdauung mit NcoI und
5) Ligation mit T4 Polynucleotidligase zum circulären Vektormolekül pGFY221N.

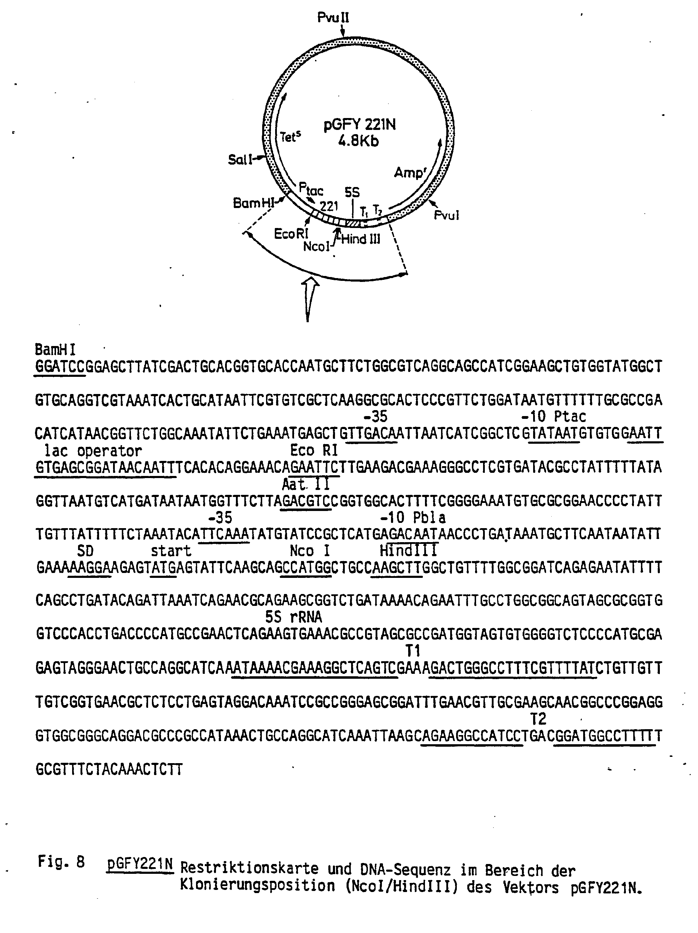

Fig. 8 zeigt die Sequenz um den interessierenden Klonierungsbereich und die Restriktionskarte des Plasmids pGFY221N. "Upstream" von der Klonierungsstelle befindet sich der tac-Promoter und "downstream" die rrnB Terminatoren T1 und T2.

Zur Integration der DNA-Kassetten A, AB und AHB wurden die Plasmide pKK-A, pKK-AB und pKK-AHB jeweils einem Doppelverdau mit NcoI und HindIII unterworfen, mit Alkalischer Phosphatase dephosphoryliert und die Vektor-DNA mit HinfI (das auf den DNA-Kassetten keine Erkennungssequenz findet) abgebaut. Die A-und AB-Kassetten wurden ohne weitere Aufreinigung in den mit NcoI/HindIII verdauten Vektor pGFY221N hineinligiert. Die AHB-Kassette wurde zunächst durch Gelelektrophorese an 5 % Polyacrylamid gereinigt und dann in pGFY221N, wie oben beschreiben, integriert. pGFY221N/AF⁻B wurde durch positionsspezifische Mutagenese von pGFY221N/AB erhalten. Dazu wurde pGFY221N/AB mit NcoI/KpnI verdaut (vergl. Fig. 1) und danach ein 13-mer und 5-mer mit kohäsiven Enden für NcoI und

5' CATGGTTTGGTAC

3' CAAAC

KpnI einligiert und damit das Codon für Phenylalanin entfernt. Dieses Verfahren der Entfernung des Codewortes für Phenylalanin ist ein Beispiel für das allgemein anwendbare Verfahren der "Kassetten-Mutagenese". Auf dem gleichen Wege können Kassetten erhalten werden, denen das Codewort für entweder Trp, Tyr, Cys oder von anderen Aminosäuren fehlt.

Damit liegen die Plasmide

pGFY221N/A

pGFY221N/AB
pGFY221N/AF⁻B und
pGFY221N/AHB vor.

Die Richtigkeit der Sequenz der jeweiligen DNA-Kassetten und des Integrationsortes (sowie des Leserasters der Proteinbiosynthese) wurde durch DNA-Sequenzierung nach Maxam und Gilbert bestätigt. Rekombinante Proteine die vom Vektor pGFY221N codiert wurden, enthalten am N-terminalen Ende zusätzlich 6 Aminosäuren (4 entstammen der Penicillinase-Signalsequenz und 2 sind durch Umwandlung der PstI-in die NcoI-Stelle entstanden). Da hier ein Fusionsprotein entsteht, das über zusätzlich 6 Aminosäuren verfugt, wurden andere Vektoren entwickelt, die eine direkte Expression der in den jeweiligen DNA-Kassetten codierten Proteininformation bewirken sollten.

Diese neuen Vektoren vom Typ pBI052 wurden aus den pGFY221N-Vektoren entwickelt.

Dazu wurden die Vektoren

pGFY221N
pGFY221N/A
pGFY221N/AB
pGFY221N/AF⁻B
pGFY221N/AHB

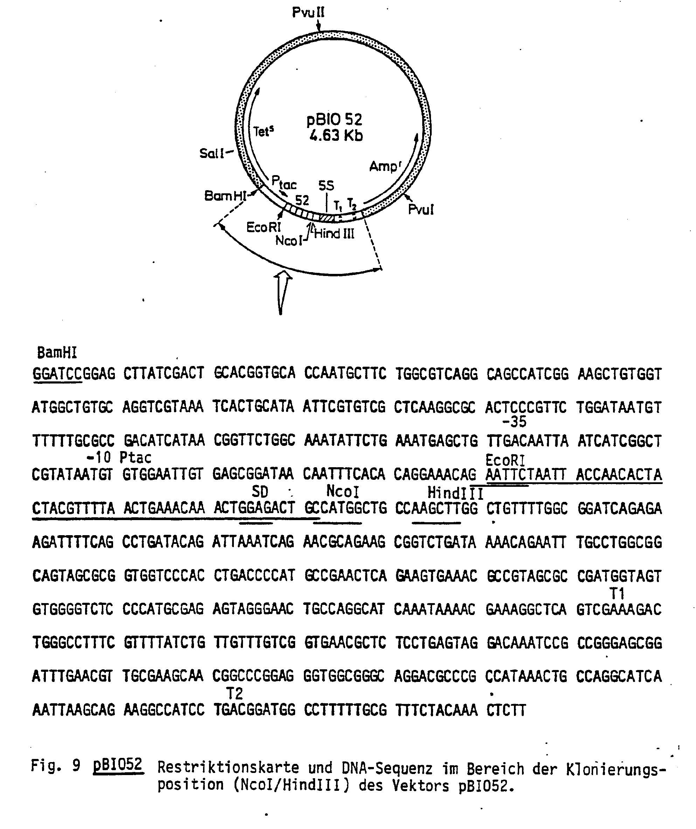

mit NcoI/EcoRI verdaut und damit ein 221 Basenpaare langes Fragment, das den β-Lactamase-Promoter und die überzähligen 6 N-terminalen Codons enthält (s. Fig. 8 und 9), entfernt. Die jeweiligen etwa 4.6 Dalton großen Plasmidfragmente wurden auf einem 1 % Agarosegel gereinigt und durch Ligation mit einem chemisch synthetisierten 52 Basenpaare langen Fragment, das für die Translations-Initiationsregion des E.coli atpE-Gens unter cyclisierenden Bedingungen mit T4 Polynucleotidligase ligiert.

Die Sequenz in der Klonierungsregion und die Restriktionskarte des neuen Plasmids pBI052 zeigt Fig. 9. Damit standen die folgenden weiteren neuen Expressionsvektoren zur Verfügung:

pBI052
pBI052/A
pBI052/AB
pBI052/AF⁻B
pBI052/AHB

Die DNA-Sequenz der jeweiligen DNA-Kassetten und die Richtigkeit des Integrationsortes wurden durch Maxam-Gilbert DNA-Sequenzierung bestätigt. Tabelle 4 zeigt die Molekulargewichte der von den pGFY221N-und pBI052-Derivaten codierten artifiziellen Proteine.

## TABELLE 4

| Artifizielles Protein | Molekulargewicht der expremierten Proteine aus pBIO 52 | Molekulargewicht der expremierten Proteine aus pGFY 221 |
|---|---|---|
| p-A | 3338,3 D | 4048,1 D (1-A)$^{*}$ |
| p-AB | 5764,8 D | 6474,6 D (1-A)$^{*}$ |
| p-AF$^{-}$B | 5599,6 D | 6309,4 D (1-AF$^{-}$B)$^{*}$ |
| p-AHB | 7648,8 D | 8358,6 D (1-AHB)$^{*}$ |
| p-A$_{6}$ | 20961,3 D | |

*)
1- Leadersequenz aus 6 Aminosäuren (Met-Ser-Ile-Gln-Ala-Ala), codiert "upstream" der einzigen Nco I Restriktionsstelle in pGFY 221N. Dieses Leaderpeptid erhöht das Molekulargewicht jedes Fusionsproteins um 638,8 D.

Tabelle 5 gibt eine Übersicht über die Proteinsequenzen der jeweiligen Proteine.

TABELLE 5

| PROTEIN | AMINOSÄURESEQUENZ |
|---|---|
| A | MVFWYLVIKVIRLRLTHKHTLIT |
| 1-A | MSIQAA MVFWYLVIKVIRLRLTHKHTLIT |
| AF⁻B | MV WYLVIKVIRLRLTHKHTLIT PGSAASADDAGPPGAEAAEAAGAC |
| 1-AF⁻B | MSIQAA MV WYLVIKVIRLRLTHKHTLIT PGSAASADDAGPPGAEAAEAAGAC |
| AB | MVFWYLVIKVIRLRLTHKHTLIT PGSAASADDAGPPGAEAAEAAGAC |
| 1-AB | MSIQAA MVFWYLVIKVIRLRLTHKHTLIT PGSAASADDAGPPGAEAAEAAGAC |
| AHB | MVFWYLVIKVIRLRLTHKHTLIT VVVVLLVIVLILILI PGSAASADDAGPPGAEAAEAAGAC |
| 1-AHB | MSIQAA MVFWYLVIKVIRLRLTHKHTLIT VVVVLLVIVLILILI PGSAASADDAGPPGAEAAEAAGAC |
| A6 | MVFWYLVIKVIRLRLTHKHTLI LR MVFWYLVIKVIRLRLTHKHTLI LR<br>MVFWYLVIKVIRLRLTHKHTLI LR MVFWYLVIKVIRLRLTHKHTLI LR<br>MVFWYLVIKVIRLRLTHKHTLI LR MVFWYLVIKVIRLRLTHKHTLIT |

In beiden Tabellen, 4 und 5, sind ebenfalls Daten enthalten, die dem durch Oligomerisierung der A-Kassette gewonnenen Hexameren A₆ entsprechen.

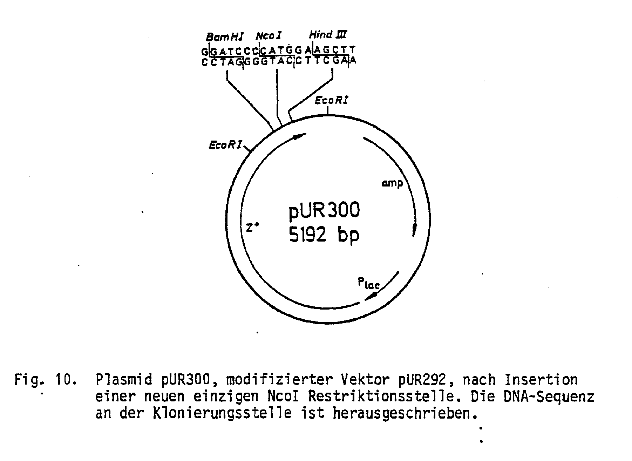

Als weiteres Expressionssystem wurden Derivate des bekannten Plasmids pUR292 hergestellt (U. Rüther und B. Müller-Hill (1983), EMBO Journal 2, 1791), mit denen ein Fusionsprotein zwischen dem größten Teil der B-Galactosidase und den artifiziellen Proteinen A, AB, AF⁻B, AHB und A₆ erhalten werden, wobei zur Stabilisierung und Schutz vor Proteasen die artifiziellen Proteine am C-terminalen Ende der β-Galaktosidase positioniert werden. Um den erfindungsgemäß geeigneten Vektor pUR300 zu erhalten, wurde das Plasmid pUR292 mit BamHI/HindIII verdaut und durch Ligation von zwei chemisch synthetisierten 12-meren, die einen doppelsträngigen Adapter mit kohäsiven BamHI-und Hind III-Enden bilden, eine zusätzliche NcoI-Stelle eingeführt. Die Vektorkonstruktion mit der DNA-Sequenz an der Klonierungsstelle zeigt Fig. 10.

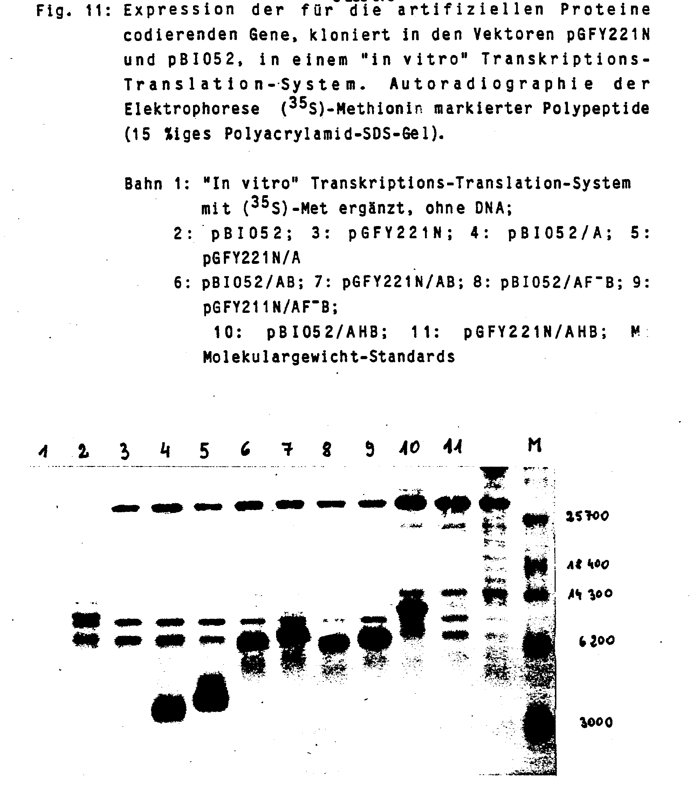

Zur in-vitro Expression wurden die entsprechend durch Ultrazentrifugation gereinigten Plasmide vom Typ pGFY221N, pBI052 und pUR300 mit einem von der Fa. Amersham erhältlichen Transkriptions-Translations-Systems in Gegenwart von entweder ³⁵S-Methionin oder ³⁵S-Cystein exprimiert und die Produkte auf einem 15 %igem SDS-Polyacrylamid-Gel durch Elektrophorese analysiert. Fig. 11 zeigt durch einen Vergleich mit Protein-Längenstandards, daß die von den DNA-Kassetten codierten Proteine A, AB, AF⁻B und AHB in den jeweiligen Plasmiden vom Typ pGFY221N und pBI052 in richtiger Größe exprimiert werden. Die Intensität der jeweiligen Proteinbanden demonstriert auch die enorm hohe in-vitro-Expressionseffizienz der artifiziellen Proteine. Da das B-Protein am C-terminalen Ende den einzigen Cystein-Baustein enthält (s. Fig. 1), kann die Intaktheit der exprimierten Proteine, die das B-Protein am C-terminalen Ende enthalten, geprüft werden.

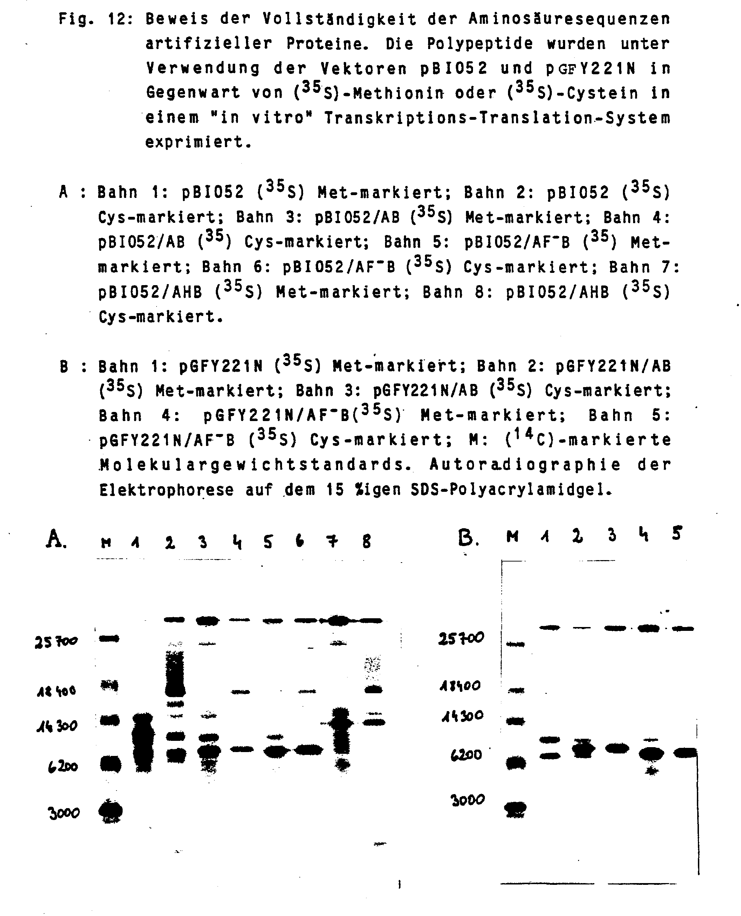

Fig. 12 zeigt, daß die Proteine p-AB, p-AF⁻B und p-AHB in Vektor pGFY221N und pBI052 in intakter Form exprimiert werden.

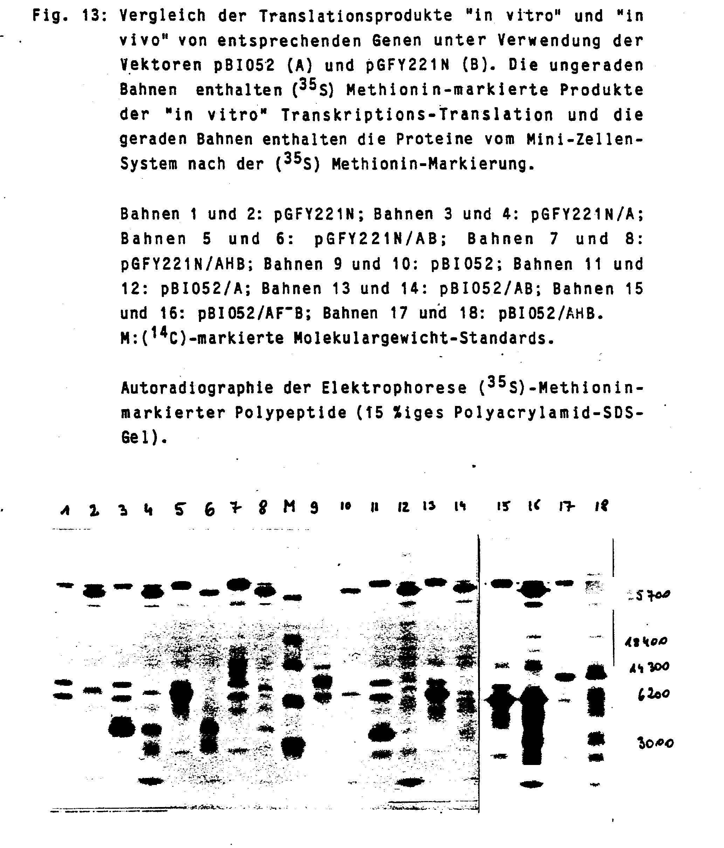

Expressionsversuch in vivo wurden im Minizell-System durchgeführt (Wirt: E. Coli CMK 603). Fig. 13 zeigt exemplarisch einige Ergebnisse. Es wird deutlich, daß die Expressionseffizienz bzw. Stabilität vom jeweiligen Vektor und dierten Protein abhängig ist. p-A wird mit dem Vektor pFGY221N, die Proteine p-AF⁻B und p-AHB mit dem Vektor pBI052 in signifikanten Aubeuten erhalten.

Es konnte durch $^{35}$S-Cystein-Markierung gezeigt werden, daß die Proteine p-AF⁻B und p-AHB auch in vivo in intakter Form, d.h. mit dem N-terminalen Methionin und dem C-terminalen Cystein exprimiert und von den E. coli-Zellen produziert werden. Tabelle 6 gibt einen Überblick über die gefundenen Proteinstabilitäten im pBI052-und pGFY221N-Vektor.

TABELLE 6

| Protein | Stabilität in Minizellen | Protein | Stabilität in Minizellen |
|---------|--------------------------|---------|--------------------------|
| p-A     | -                        | 1-A     | ++                       |
| p-AB    | -                        | 1-A?    | +                        |
| p-AHB   | ++                       | 1-AHB   | +                        |

```
++  vollständig stabil
+   teilweise stabil
-   nicht stabil
```

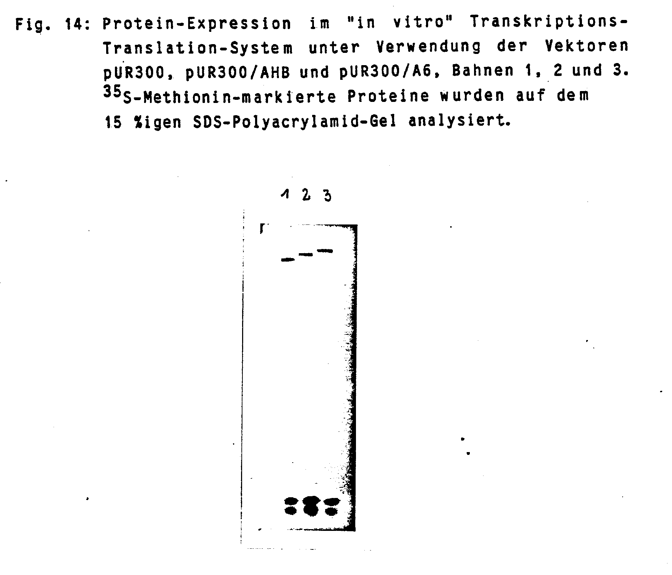

Als weiters Beispiel für die Expression der artifiziellen Proteine wurden die DNA-Kassetten für die Proteine p-AHB und p-A₆ im Vektor pUR300 exprimiert. Hier werden die Proteine im C-terminalen Bereich der β-Galaktosidase anfusioniert. Als Wirt wurde der lac-Repressor überproduzierende E.coli-Stamm BMH 71-18 verwendet. Durch IPTG kann die Expression des lac-Operons individuell induziert werden. Fig. 14 zeigt die in-vitro Expression der Fusionsproteine nach Markierung mit $^{35}$S-Methionin.

Man erkennt deutlich, daß in sehr sauberer Reaktion entsprechend verlängerte Proteine exprimiert und produziert werden.

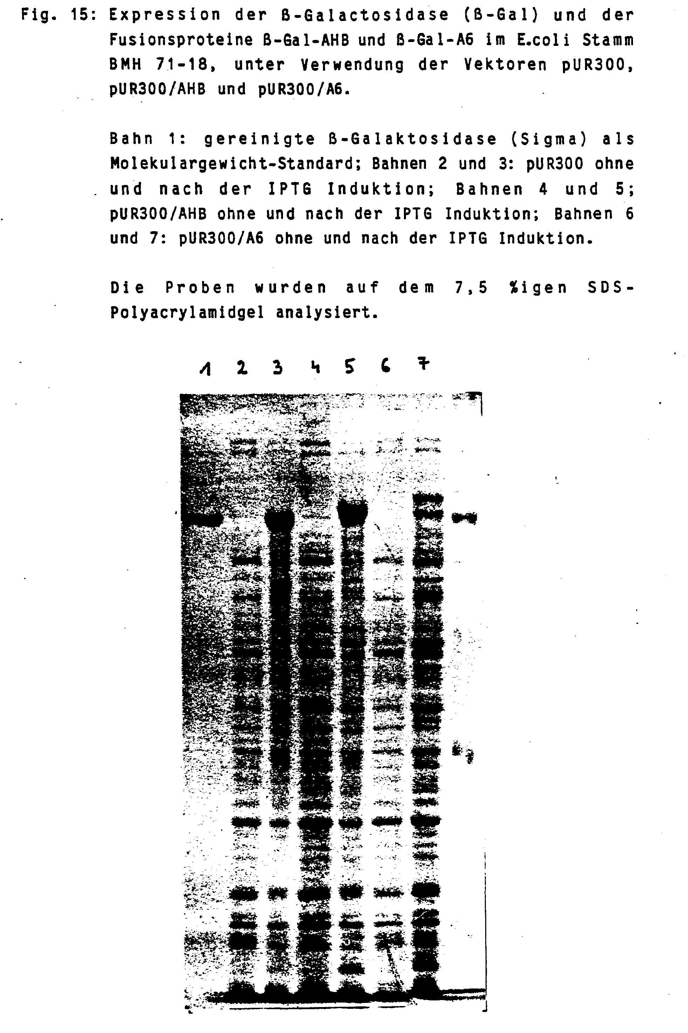

Fig. 15 zeigt die Produktion der Fusionsproteine in-vivo im E.coli-Stamm BMH 71-18. Neben der Synthese anderer E.coli-Proteine ist deutlich die Überproduktion der gewünschten Fusionsproteine zu erkennen. Da die Synthese allein durch IPTG induzierbar ist, handelt es sich eindeutig um Produkte der lac-Operon-Expression. Die Fusionsproteine konnten durch Affinitätschromatographie nach üblichen Methoden gereinigt werden.

Neben den detailliert beschriebenen Plasmiden pGFY221N, pBI052 und pUR300 eignen sich zur Integration der Kassetten A, AF⁻, B und H, von deren Mutageneseprodukten mit einer Reduktion von maximal 4 Codewörtern pro Kassette, von deren Oligomeren oder von deren Kombinationen, auch noch andere bekannte Expressionsvektor/Wirtssysteme. Die jeweiligen, geeigneten Expressionsvektoren können in den homologen Prokaryonten, z.B. die beschriebenen Bakterienstämme E.coli CMK 603, DS 410 und BMH 71-18, und Eukaryonten, z.B. Hefen, einschließlich Pflanzen transformiert werden.

Experimentelle Beispiele sind oben für die Proteine p-A, p-AB, p-AF⁻B, p-A₆ und p-AHB im De schrieben worden. Hierbei wurde auf im allgemeinen bekannte Verfahren zurückgegriffen. Die chemische Synthese der einzelsträngigen DNA-Fragmente erfolgte nach Köster et al. (1984), Nucleic Acids Res. 12, 4539. DNA-Präparationen, Restriktions-und Ligationsversuche, radioaktives Markieren von DNA durch Klenow-Enzym, Agarose-und Polyacrylamidgelelektrophorese, Plasmidkonstruktionen, Transformationen von E.coli-Zellen, in vitro Expressions-Experimente und die Verwendung von E.coli-Mini-Zellen erfolgen nach Verfahren wie sie in der Literatur beschrieben sind, wie z.B. in T. Maniatis et al. (1982) "Molecular Cloning: A Laboratory Manual", Cold Spring Harbor Laboratory, New York, and J. Brosius (1986) in "Vectors: A Summary of Molecular Cloning Vectors and Their Uses" (R.C. Rodriguez and D.T. Denhardt, Herausgeber; Butterworth Publishers, Stoneham, Mass., U.S.A.), sowie N.G. Stoker et al. (1984) in "Transcription and Translation. A practical approach", (B.D. Hannes und S.J. Higgins, Herausgeber; IRL Press, Oxford, U.K.). Ferner J.H. Miller (1972) "Experiments in Molecular Genetics", Cold Spring Harbor Laboratory, New York. Die DNA-Sequenzierung erfolgte nach A.M. Maxam und W. Gilbert (1980), Methods Enzymol. 65, 499.

Bei der Deutschen Sammlung von Mikroorganismen, Grisebachstr. 8, D-3400 Göttingen, sind in Übereinstimmung mit dem Budapester Vertrag folgende erfindungsgemäß benutzte Wirt-Vektor-Systeme hinterlegt

1.) E. coli CMK 603 enthaltend das Plasmid pGFY221N mit Kassette A

2.) E. Coli CMK 603 enthaltend das Plasmid pBI052 mit Kassette AF⁻B

3.) E. coli CMK 603 enthaltend das Plasmid pBI052 mit Kassette AHB

4.) E. coli BMH 71-18 enthaltend das Plasmid pUR300 mit Kassette A₆

**Ansprüche**

1. Synthetische DNA-Kassette mit Codierung für ein artifizielles Protein vorgegebener Aminosäurezusammensetzung

2. DNA-Kassette A folgender Nucleotidsequenz:
GGCCATGGTTTTCTGGTACCTGGTTATCAAAGTTATCCGTCTGCGTCTGACCCACAAACACAC
CCGGTACCAAAAGACCATGGACCAATAGTTTCAATAGGCAGACGCAGACTGGGTGTTTGTGTG

CCTGATCACCTGAAGCTTGG
GGACTAGTGGACTTCGAACC

3. DNA-Kassette B folgender Nucleotidsequenz:
CCCTGATCACCCCGGGTTCCGCGGCTTCCGCAGACGACGCAGGTCCGCCGGGTGCTGAGGCAG
GGGACTAGTGGGGCCCAAGGCGCCGAAGGCGTCTGCTGCGTCCAGGCGGCCCACGACTCCGTC

CTGAGGCTGCAGGTGCATGCTGAAGCTTGG
GACTCCGACGTCCACGTACGACTTCGAACC

4. DNA-Kassette H folgender Nucleotidsequenz:
GCCTGATCACCGTTGTTGTGGTTCTGCTGGTTATCGTGCTGATCCTGATGCTGATCCCGGGCCG
CGGACTAGTGGCAACAACACCAAGACGACCAATAGCACGACTAGGACTACGACTAGGGCCCGGC

5. DNA-Kassette nach einem der Ansprüche 1 bis 4, dadurch gekennzeichnet, daß sie durch Aneinanderkoppeln oligomerisiert ist, wobei der Oligomerisierungsgrad m 2 bis 12 ist.

6. DNA-Kassette nach einem der Ansprüche 1 bis 5 in mutagenisierter Form mit einer Reduktion von maximal 4 Codewörtern pro Kassette.

7. Kombination der DNA-Kassetten A, B und H nach Ansprüchen 1, 2, 3, 4, 5 und/oder 6.

8. Expressionsvektor, dadurch gekennzeichnet, daß er eine gegebenenfalls oligomerisierte und/oder mutagenisierte DNA-Kassette A nach einem der Ansprüche 2, 5 oder 6 enthält.

9. Expressionsvektor, dadurch gekennzeichnet, daß er eine gegebenenfalls oligomerisierte und/oder mutagenisierte DNA-Kassette B nach einem der Ansprüche 3, 5 oder 6 enthält.

10. Expressionsvektor, dadurch gekennzeichnet, daß er eine gegebenenfalls oligomerisierte und/oder mutagenisierte DNA-Kassette H nach einem der Ansprüche 4, 5 oder 6 enthält.

11. Expressionsvektor, dadurch gekennzeichnet, daß er eine Kombination von DNA-Kassetten nach Anspruch 7 enthält.

12. Expressionsvektor nach Anspruch 8, dadurch gekennzeichnet, daß er eine der folgenden mutagenisierten Kassetten A enthält:
$AF^-$, $AW^-$, $AY^-$, $AM^-$.

13. Expressionsvektor nach Anspruch 11, dadurch gekennzeichnet, daß er eine Kombination folgender Kassetten enthält:
AB, $AF^-B$, $AW^-B$, $AY^-B$, $AM^-B$, $ABC^-$, AHB, $AW^-HB$, $AHBC^-$, $A_2B$, $A_6B$, $A_{12}B$, $AB_2$, $AB_6$, $AB_{12}$, $AHB_2$ oder $AHB/AW^-HB$.

14. Expressionsvektor nach einem der Ansprüche 8 bis 13, dadurch gekennzeichnet, daß er ein Plasmid ist.

15. Plasmid pGFY221N

16. Plasmid pBI052

17. Plasmid pUR300

18. Plasmid pGFY221N enthaltend eine gegebenenfalls oligomerisierte und/oder mutagenisierte DNA-Kassette nach einem der Ansprüche 2 bis 6 oder eine Kassetten-Kombination nach Anspruch 7.

19. Plasmid pBI052 enthaltend eine gegebenenfalls oligomerisierte und/oder mutagenisierte DNA-Kassette nach einem der Ansprüche 2 bis 6 oder eine Kassettenkombination nach Anspruch 7.

20. Plasmid pUR300 enthaltend eine gegebenenfalls oligomerisierte und/oder mutagenisierte DNA-Kassette nach einem der Ansprüche 2 bis 6 oder eine Kassetten-Kombination nach Anspruch 7.

21. Plasmid pGFY221N enthaltend die DNA-Kassette A

22. Plasmid pGFY221N enthaltend die DNA-Kassetten AB

23. Plasmid pGFY221N enthaltend die DNA-Kassetten $AF^-B$

24. Plasmid pGFY221N enthaltend die DNA-Kassetten AHB

25. Plasmid pBI052 enthaltend die DNA-Kassette A

26. Plasmid pBI052 enthaltend die DNA-Kassetten AB

27. Plasmid pBI052 enthaltend die DNA-Kassetten $AF^-B$

28. Plasmid pBI052 enthaltend die DNA-Kassetten AHB

29. Plasmid pUR300 enthaltend die DNA-Kassetten AHB

30. Plasmid pUR300 enthaltend die oligomerisierte DNA-Kassette A₆

31. Wirt-Vektor-System, bestehend aus einem Pro-oder Eukaryonten einschließlich einer Pflanze und einem transformierten Expressionsvektor nach einem der Ansprüche 8 bis 30.

32. E. coli CMK 603 enthaltend das Plasmid pGFY221N

33. E. coli CMK 603 enthaltend das Plasmid pBI052

34. E. coli CMK 603 enthaltend das Plasmid nach Anspruch 21

35. E. coli CMK 603 enthaltend das Plasmid nach Anspruch 22

36. E. coli CMK 603 enthaltend das Plasmid nach Anspruch 23

37. E. coli CMK 603 enthaltend das Plasmid nach Anspruch 24

38. E. coli CMK 603 enthaltend das Plasmid nach Anspruch 25

39. E. coli CMK 603 enthaltend das Plasmid nach Anspruch 26

40. E. coli CMK 603 enthaltend das Plasmid nach Anspruch 27

41. E. coli CMK 603 enthaltend das Plasmid nach Anspruch 28

42. E. coli DS 410 enthaltend das Plasmid pGFY221N

43. E. coli DS 410 enthaltend das Plasmid pBI052

44. E. coli DS 410 enthaltend das Plasmid nach Anspruch 21

45. E. coli DS 410 enthaltend das Plasmid nach Anspruch 22

46. E. coli DS 410 enthaltend das Plasmid nach Anspruch 24

47. E. coli DS 410 enthaltend das Plasmid nach Anspruch 25

48. E. coli DS 410 enthaltend das Plasmid nach Anspruch 26

49. E. coli DS 410 enthaltend das Plasmid nach Anspruch 27

50. E. coli DS 410 enthaltend das Plasmid nach Anspruch 28

51. E. coli BMH 71-18 enthaltend das Plasmid pUR300

52. E. coli BMH 71-18 enthaltend das Plasmid nach Anspruch 29

53. E. coli BMH 71-18 enthaltend das Plasmid nach Anspruch 30

54. Artifizielles Protein, codiert durch eine DNA-Kassette nach einem der Ansprüche 1 bis 6 oder eine Kassetten-Kombination gemäß Anspruch 7.

55. Artifizielles Protein nach Anspruch 54, codiert durch die DNA-Kassette A nach Anspruch 2, enthaltend 13,0 Mol-% Ile, 17,4 Mol-% Leu, 13,0 Mol-% Val, 8,7 Mol-% Lys, 4,3 Mol-% Met, 13,0 Mol-% Thr, 4,3 Mol-% Trp, 4,3 Mol-% Tyr, 8,7 Mol-% Arg und 8,7 Mol-% His.

56. Artifizielles Protein nach Anspruch 54, codiert durch eine mutagenisierte DNA-Kassette nach Anspruch 6, ent haltend 13,6 Mol-% Ile, 18,2 Mol-% Leu, 13,6 Mol-% Val, 9,1 Mol-% Lys, 13,6 Mol-% Thr, 9,1 Mol-% Arg und 9,1 Mol-% His sowie entweder jeweils 4,5 Mol-% Met, Trp und Tyr (Kassette AF⁻) oder jeweils 4,5 Mol-% Met, Phe und Tyr (Kassette AW⁻) oder jeweils 4,5 Mol-% Met, Phe und Trp (Kassette AY⁻) oder jeweils 4,5 Mol-% Phe, Trp und Tyr (Kassette AM⁻).

57. Artifizielles Protein nach Anspruch 54, codiert durch eine Kombination der DNA-Kassetten A und B (Kassette AB) nach Anspruch 7, enthaltend 6,4 Mol-% Ile, 8,5 Mol-% Leu, 6,4 Mol-% Val, 4,3 Mol-% Lys, 2,1 Mol-% Met, 2,1 Mol-% Phe, 6,4 Mol-% Thr, 2,1 Mol-% Trp, 2,1 Mol-% Tyr, 4,3 Mol-% Arg, 4,3 Mol-% His, 21,3 Mol-% Ala, 4,3 Mol-% Asp, 2,1 Mol-% Cys, 4,3 Mol-% Glu, 8,5 Mol-% Gly, 6,4 Mol-% Pro und 4,3 Mol-% Ser.

58. Artifizielles Protein nach Anspruch 54, codiert durch eine Kombination einer mutagenisierten DNA-Kassette A mit der DNA-Kassette B nach Anspruch 7, enthaltend 6,5 Mol-% Ile, 8,7 Mol-% Leu, 6,5 Mol-% Val, 4,3 Mol-% Lys, 6,5 Mol-% Thr, 4,3 Mol-% Arg und 4,3 Mol-% His und entweder jeweils 2,2 Mol-% Met, Trp und Tyr (Kassetten-Kombination AF⁻B) oder jeweils 2,2 Mol-% Met, Phe und Tyr (Kassetten-Kombination AW⁻B) oder jeweils 2,2 Mol-% Met, Phe und Trp (Kassetten-Kombination AY⁻B) oder jeweils 2,2 Mol-% Phe, Trp und Tyr (Kassetten-Kombination AM⁻B).

59. Artifizielles Protein nach Anspruch 54, codiert durch eine Kombination der DNA-Kassette A mit einer mutagenisierten DNA-Kassette B (Kassette ABC⁻) nach Anspruch 7, enthaltend 6,5 Mol-% Ile, 8,7 Mol-% Leu, 6,5 Mol-% Val, 4,3 Mol-% Lys, 2,2 Mol-% Met, 2,2 Mol-% Phe, 6,5 Mol-% Thr, 2,2 Mol-% Trp, 2,2 Mol-% Tyr, 4,3 Mol-% Arg, 4,3 Mol-% His, 21,7 Mol-% Ala, 4,3 Mol-% Asp, 4,3 Mol-% Glu, 8,7 Mol-% Gly, 6,5 Mol-% Pro und 4,3 Mol-% Ser.

60. Artifizielles Protein nach Anspruch 57, codiert durch eine Kombination der DNA-Kassetten A, B und H nach Anspruch 7, enthaltend 11,3 Mol-% Ile, 14,5 Mol-% Leu, 14,5 Mol-% Val, 3,2 Mol-% Lys, 1,6 Mol-% Met, 1,6 Mol-% Phe, 4,8 Mol-% Thr, 1,6 Mol-% Trp, 1,6 Mol-% Tyr, 3,2 Mol-% Arg, 3,2 Mol-% His, 16,1 Mol-% Ala, 3,2 Mol-% Asp, 1,6 Mol-% Cys, 3,2 Mol-% Glu, 6,5 Mol-% Gly, 4,8 Mol-% Pro und 3,2 Mol-% Ser.

61. Artifizielles Protein nach Anspruch 54, codiert durch eine Kombination einer mutagenisierten DNA-Kassette A mit den DNA-Kassetten .B und H (Kassetten-Kombination $AW^-HB$) nach Anspruch 7, enthaltend 11,5 Mol-% Ile, 14,8 Mol-% Leu, 14,8 Mol-% Val, 3,3 Mol-% Lys, 1,6 Mol-% Met, 1,6 Mol-% Phe, 4,9 Mol-% Thr, 1,6 Mol-% Tyr, 3,3 Mol-% Arg, 3,3 Mol-% His, 16,4 Mol-% Ala, 3,3 Mol-% Asp, 1,6 Mol-% Cys, 3,3 Mol-% Glu, 6,6 Mol-% Gly, 4,9 Mol-% Pro und 3,3 Mol-% Ser.

62. Artifizielles Protein nach Anspruch 54, codiert durch eine Kombination der DNA-Kassetten A und H mit einer mutagenisierten DNA-Kassette B (Kassetten-Kombination $AHBC^-$) nach Anspruch 7, enthaltend 11,5 Mol-% Ile, 14,8 Mol-% Leu, 14,8 Mol-% Val, 3,3 Mol-% Lys, 1,6 Mol-% Met, 1,6 Mol-% Phe, 4,9 Mol-% Thr, 1,6 Mol-% Trp, 1,6 Mol-% Tyr, 3,3 Mol-% Arg, 3,3 Mol-% His, 16,4 Mol-% Ala, 3,3 Mol-% Asp, 3,3 Mol-% Glu, 6,6 Mol-% Gly, 4,9 Mol-% Pro und 3,3 Mol-% Ser.

63. Artifizielles Protein nach Anspruch 54, codiert durch eine oligomerisierte (m = 6) DNA-Kassette A, das die in Anspruch 58 angegebene Aminosäurezusammensetzung aufweist und ein Molekulargewicht von 20961, 3 D besitzt.

64. Artifizielles Protein nach Anspruch 54, codiert durch eine Kombination oligomerisierter (m = 2, 6 bzw. 12) DNA-Kassetten A mit der DNA-Kassette B nach Anspruch 7, enthaltend 8,6 Mol-% Ile, 11,4 Mol-% Leu, 8,6 Mol-% Val, 5,8 Mol-% Lys, 2,8 Mol-% Met, 2,8 Mol-% Phe, 8,6 Mol-% Thr, 2,8 Mol-% Trp, 2,8 Mol-% Tyr, 5,8 Mol-% Arg, 5,8 Mol-% His, 14,3 Mol-% Ala, 2,9 Mol-% Asp, 1,4 Mol-% Cys, 2,9 Mol-% Glu, 5,7 Mol-% Gly, 4,3 Mol-% Pro und 2,9 Mol-% Ser (Kassette $A_2B$); 11,1 Mol-% Ile, 14,8 Mol-% Leu, 11,1 Mol-% Val, 7,5 Mol-% Lys, 3,7 Mol-% Met, 3,7 Mol-% Phe, 11,1 Mol-% Thr, 3,7 Mol-% Trp, 3,7 Mol-% Tyr, 7,5 Mol-% Arg, 7,5 Mol-% His, 6,2 Mol-% Ala, 1,2 Mol-% Asp, 0,6 Mol-% Cys, 1,2 Mol-% Glu, 2,5 Mol-% Gly, 1,9 Mol-% Pro und 1,2 Mol-% Ser (Kassette $A_6B$) bzw. 12,0 Mol-% Ile, 16,0 Mol-% Leu, 12,0 Mol-% Val, 8,1 Mol-% Lys, 3,9 Mol-% Met, 3,9 Mol-% Phe, 12,0 Mol-% Thr, 3,9 Mol-% Trp, 3,9 Mol-% Tyr, 8,1 Mol-% Arg, 8,1 Mol-% His, 3,3 Mol-% Ala, 3,7 Mol-% Asp, 0,3 Mol-% Cys, 0,7 Mol-% Glu, 1,3 Mol-% Gly, 1,0 Mol-% Pro und 0,7 Mol-% Ser (Kassette $A_{12}B$).

65. Artifizielles Protein nach Anspruch 54, codiert durch eine Kombination der DNA-Kassette A mit einer oligomerisierten (m = 2, 6 bzw. 12) DNA-Kassette B nach Anspruch 7, enthaltend 4,2 Mol-% Ile, 5,6 Mol-% Leu, 4,2 Mol-% Val, 2,8 Mol-% Lys, 1,4 Mol-% Met, 1,4 Mol-% Phe, 4,2 Mol-% Thr, 1,4 Mol-% Trp, 1,4 Mol-% Tyr, 2,8 Mol-% Arg, 2,8 Mol-% His, 28,1 Mol-% Ala, 5,7 Mol-% Asp, 2,8 Mol-% Cys, 5,7 Mol-% Glu, 11,2 Mol-% Gly, 8,5 Mol-% Pro und 5,7 Mol-% Ser (Kassette $AB_2$); 1,8 Mol-% Ile, 2,4 Mol-% Leu, 1,8 Mol-% Val, 1,2 Mol-% Lys, 0,6 Mol-% Met, 0,6 Mol-% Phe, 1,8 Mol-% Thr, 0,6 Mol-% Trp, 0,6 Mol-% Tyr, 1,2 Mol-% Arg, 1,2 Mol-% His, 35,9 Mol-% Ala, 7,2 Mol-% Asp, 3,5 Mol-% Cys, 7,3 Mol-% Glu, 14,3 Mol-% Gly, 10,8 Mol-% Pro und 7,2 Mol-% Ser (Kassette $AB_6$) bzw. 1,0 Mol-% Ile, 1,3 Mol-% Leu, 1,0 Mol-% Val, 0,7 Mol-% Lys, 0,3 Mol-% Met, 0,3 Mol-% Phe, 1,0 Mol-% Thr, 0,3 Mol-% Trp, 0,3 Mol-% Tyr, 0,7 Mol-% Arg, 0,7 Mol-% His, 38,5 Mol-% Ala, 7,8 Mol-% Asp, 3,8 Mol-% Cys, 7,8 Mol-% Glu, 15,4 Mol-% Gly, 11,6 Mol-% Pro und 7,8 Mol-% Ser (Kassette $AB_{12}$).

66. Artifizielles Protein nach Anspruch 54 codiert durch eine Kombination der DNA-Kassetten A und H mit einer oligomerisierten (m = 2) DNA-Kassette B (Kassette $AHB_2$) nach Anspruch 7, enthaltend 8,2 Mol-% Ile, 10,5 Mol-% Leu, 8,2 Mol-% Val, 2,3 Mol-% Lys, 1,2 Mol-% Met, 1,2 Mol-% Phe, 3,5 Mol-% Thr, 1,2 Mol-% Trp, 1,2 Mol-% Tyr, 2,3 Mol-% Arg, 2,3 Mol-% His, 23,3 Mol-% Ala, 4,6 Mol-% Asp, 2,3 Mol-% Cys, 4,6 Mol-% Glu, 9,4 Mol-% Gly, 6,9 Mol-% Pro und 4,6 Mol-% Ser.

67. Artizielles Protein nach Anspruch 54 codiert durch eine Kombination der DNA-Kassetten A, H und B einerseits und eine Kombination einer mutagenisierten DNA-Kassette A mit den DNA-Kassetten B und H (Kassetten-Kombination $AHB/AW^-HB$) nach Anspruch 7, enthaltend 11,4 Mol-% Ile, 14,7 Mol-% Leu, 14,7 Mol-% Val, 3,2 Mol-% Lys, 1,6 Mol-% Met, 1,6 Mol-% Phe, 4,9 Mol-% Thr, 0,8 Mol-% Trp, 1,6 Mol-% Tyr, 3,2 Mol-% Arg, 3,2 Mol-% His, 16,3 Mol-% Ala, 3,2 Mol-% Asp, 1,6 Mol-% Cys, 3,2 Mol-% Glu, 6,6 Mol-% Gly, 4,9 Mol-% Pro und 3,2 Mol-% Ser.

68. Verfahren zur Herstellung von Proteinen nach einem der Ansprüche 54 bis 67 auf gentechnologischem Wege, dadurch gekennzeichnet, daß man in an sich bekannter Weise mindestens eine synthetische DNA-Kassette nach Anspruch 1 mit Codierung für ein artifizielles Protein vorgegebener Aminosäurezusammensetzung synthetisiert, in einen zur Expression geeigneten Vektor integriert diesen in einen geeigneten Wirt transformiert und dort unter geeigneten Bedingungen zur Expression bringt.

69. Verfahren nach Anspruch 68 zur Herstellung eines Proteins nach Anspruch 54, dadurch gekennzeichnet, daß man in den Vektor eine DNA-Kassette nach einem der Ansprüche 2 bis 6 oder eine Kassetten-Kombination nach Anspruch 7 integriert.

70. Verfahren nach Anspruch 68 zur Herstellung eines Proteins nach Anspruch 55, dadurch gekennzeichnet, daß man in den Vektor eine DNA-Kassette A integriert.

71. Verfahren nach Anspruch 68 zur Herstellung eines Proteins nach Anspruch 56, dadurch gekennzeichnet, daß man in den Vektor eine mutagenisierte DNA-Kassette A, nämlich $AF^-$, $AW^-$, $AY^-$ oder $AM^-$ integriert.

72. Verfahren nach Anspruch 68 zur Herstellung eines Proteins nach einem der Ansprüche 57 bis 67, dadurch gekennzeichnet daß man in den Vektor eine der folgenden Kassetten-Kombinationen AB, $AF^-B$, $AW^-B$, $AY^-B$, $AM^-B$, $ABC^-$, AHB, $AW^-HB$, $AHBC^-$, $A_2B$, $A_6B$, $A_{12}B$, $AB_2$, $AB_6$, $AB_{12}$, $AHB_2$ oder $AHB/AW^-HB$ integriert.

73. Verfahren nach einem der Ansprüche 68 bis 72, dadurch gekennzeichnet, daß man als zur Expression geeigneten Vektor ein Plasmid verwendet.

74. Verfahren nach Anspruch 73, dadurch gekennzeichnet, daß man ein Plasmid verwendet, das zu einer direkten Expression des durch die Kassette(n) codierten Proteins führt.

75. Verfahren nach Anspruch 73, dadurch gekennzeichnet, daß man ein Plasmid verwendet, das zur Expression eines Fusionsproteins aus Fremdprotein und durch die Kassette(n) codiertem Protein führt.

76. Verfahren nach einem der Ansprüche 68 bis 75, dadurch gekennzeichnet, daß man als Wirt für den transformierten Expressionsvektor einen Prokaryonten oder Eukaryonten einschließlich einer Pflanze verwendet.

77. Verfahren nach Anspruch 76, dadurch gekennzeichnet, daß man als Wirt einen E. coli-Stamm verwendet.

78. Verfahren nach Anspruch 77, dadurch gekennzeichnet, daß man als E. coli-Stamm den Stamm E. coli CMK 603, HB 101, DS 410 oder BMH 71-18 verwendet.

79. Verfahren nach Anspruch 67, dadurch gekennzeichnet, daß man ein Wirt/Vektor-System gemäß einem der Ansprüche 35 bis 43, 46 bis 53, 55 oder 56 verwendet.

80. Verfahren zur schrittweisen Oligomerisierung von DNA-Sequenzen, dadurch gekennzeichnet, daß man

A) die in ein Plasmid integrierte und zu oligomerisierende Fremd-DNA auf einer Seite am 3'-oder 5'-Ende (a oder b) schneidet (Stufe 1 von Fig. 7); an beide Enden der geöffneten Fremd-DNA Adaptermoleküle anligiert (Stufe 2 von Fig. 7), wobei die Adaptermoleküle die Spezifität der kohäsiven Enden der nicht geöffneten Seite der Fremd-DNA ohne Möglichkeit zur Restaurierung der Restriktionserkennungsstelle herstellen (Fig. 6), die Fremd-DNA am (anderen) 5'-oder 3'-Ende (b oder a) schneidet (Stufe 3a in Fig. 7), und die noch vorhandenen 5'-OH-Enden mit T4-Polynucleotidkinase phosphoryliert;

B) ein Aliquot zu der in das Plasmid integrierten und zu oligomerisierenden Fremd-DNA auf der anderen Seite schneidet (Stufe 3b von Fig. 7), und durch Behandeln mit Alkalischer Phosphatase dephosphoryliert;

C) die beiden Ansätze aus den Verfahrensstufen A) und B) miteinander vereinigt und mit Hilfe von DNA-Ligasen ligiert (Stufe 4 von Fig. 7);

D) eine Klonierung durchführt;

E) den Klon mit der dublizierten Fremd-DNA isoliert und

F) gegebenenfalls die Verfahrensstufen A) bis C) beliebig oftwiederholt.

81. Verfahren zur schrittweisen Oligomerisierung von DNA-Sequenzen, dadurch gekennzeichnet, daß man

A) die in ein Plasmid integrierte und zu oligomerisierende Fremd-DNA auf einer Seite am 3'-oder 5'-Ende (a oder b) schneidet (Stufe 1 von Fig. 7); an beide Enden der geöffneten Fremd-DNA Adaptermoleküle anligiert (Stufe 2 von Fig. 7), wobei die Adaptermoleküle die Spezifität der kohäsiven Enden der nicht geöffneten Seite der Fremd-DNA ohne Möglichkeit zur Restaurierung der Restriktionserkennungsstelle herstellen (Fig. 6), die Fremd-DNA am (anderen) 5'-oder 3'-Ende (b oder a) schneidet (Stufe 3a in Fig. 7), und die noch vorhandenen 5'-OH-Enden mit T4-Polynucleotidkinase phosphoryliert, und

B) die vom Plasmid befreite bzw. isolierte Fremd-DNA in Gegenwart der entsprechenden Restriktionsenzyme (mit entweder a-oder b-Spezifität) durch T4-Polynucleotidligase ligiert und gegebenenfalls die Schritte A) und B) wiederholt;

C) die so erhaltenen Oligomere in ein geeignetes Plasmid integriert und nach Transformation in geeignete E. coli Stämme die oligomerisierte Fremd-DNA kloniert.

## Figur 1

### Segment A (83 bp)

```
    M  V  F  W  Y  L  V  I  K  V  I  R  L  R  L  T  H  K  H  T  L  I  T
GGCCATGGTTTTCTGGTACCTGGTTATCAAAGTTATCCGTCTGCGTCTGacccacaaacacaccctgatcacctgaagcttgg
ccggtaccaaaagaccatggaccaatagtttcaatagGCAGACGCAGACTGGGTGTTTGTGTGGGACTAGTGGACTTCGAACC
    -Nco1-        -Kpn1-                                    -Bcl1-      -Hind3-
```

### Segment B (93 bp)

```
    L  I  T  P  G  S  A  A  S  A  D  D  A  G  P  P  G  A  E  A  A  E  A  A  G  A  C  *
CCCTGATCACCCCGGGTTCCGCGGCTTCCGCAGACGACGCAGGTCCGCCGGGTgctgaggcagctgaggctgcaggtgcatgctgaagcttgg
gggactagtggggcccaaggcgccgaaggcgtctgctgcgtCCAGGCGGCCCACGACTCCGTCGACTCCGACGTCCACGTACGACTTCGAACC
    -Bcl1-  -Xma1-  -Sac2-                              -Pvu2-    -Pst1-  -Sph1-  -Hind3-
```

### Segment H (64 bp)

```
    L  I  T  V  V  V  V  L  L  V  I  V  L  I  L  I  L  I  P  G
GCCTGATCACCGTTGTTGTGGTTCTGCTGGTTATCGTGctgatcctgatgctgatcccggggccg
cggactagtggcaacaacaccaagacGACCAATAGCACGACTAGGACTACGACTAGGGCCCGGC
    -Bcl1-                                        -Xma1-
```

Fig. 1    DNA-Sequenzen der DNA-Kassetten für die Proteine p-A, p-B
         und p-H durch kombinierte chemische/enzymatische DNA-Synthese.

Fig. 2: "In vitro" Synthese der doppelsträngigen Fragmente A, B und H unter Verwendung des Klenow Fragments der E.coli Polymerase I.

a) Bildungsgeschwindigkeit des voll ausgebildeten doppelsträngigen Segments A: Oligomer A-1 (49-mer), Bahn 1; Oligomer A-2 (46-mer) Bahn 2; Homo-Oligo-dT Lösungsstandard; Bahn 3; Reaktionsgemisch vor der Enzymzugabe, Bahn 4; Reaktionsgemisch 1,5; 2,5; 5; 15 und 30 Minuten nach der Enzymzugabe, Bahnen 5, 6, 7, 8 und 9.

b) Enzymatische Synthese des Segments B: Oligomer B-2 (52-mer), Bahn 1; Oligomer B-1 (53-mer), Bahn 2; Reaktionsgemisch 45 Min. nach dem Reaktionsanfang (25°C), Bahn 3.

c) Enzymatische Synthese des Segments H: Mischung der Oligomere H1 und H2 (38-mere) vor der enzymatischen Reaktion, Bahn 1; Reaktionsgemisch nach 30 Min. Reaktion, (25°C), Bahn 2.

Polyacrylamid-Gelelektrophorese in 7M Harnstoff (a), (b): 8 % Acrylamid und (c) 5 % Acrylamid.

5' A1 (49-mer) 3'
3' 12 bp A2 (46-mer) 5'

DNA polymerase I (Klenow)

NcoI     BclI   HindⅢ
A segment (83 bp)
HindⅢ
NcoI

pKK 233-2
PvuⅡ
ori
amp
BamHI
EcoRI
trc
5s t1 t2 PvuI

SD
NcoI   PstI   HindⅢ
ACAGGAAACAGACCATGGCTGCAGCCAAGCTTGGC
TGTCCTTTGTCTGGTACCGACGTCGGTTCGAACCG

HindⅢ
NcoI

5' B1 (53-mer) 3'
3' 12 bp B2 (52-mer) 5'

DNA polymerase I (Klenow)

BclI XmaI     HindⅢ
B segment (93 bp)
BclI
HindⅢ

pKK-A
PvuⅡ
ori
amp
BamHI
EcoRI
trc NcoI BclI
HindⅢ
A
PvuI

BclI
HindⅢ

5' H1 (38-mer) 3'
3' 12 bp H2 (38-mer) 5'

DNA polymerase I (Klenow)

BclI     XmaI
H segment (62 bp)
BclI
XmaI

pKK-AB
PvuⅡ
ori
amp
BamHI
EcoRI
trc NcoI HindⅢ
BclI XmaI
A B
PvuI
BclI
XmaI

pKK-AHB
PvuⅡ
ori
amp
BamHI
EcoRI
trc NcoI HindⅢ
BclI XmaI
A H B
PvuI

Fig. 3. Strategie der Klonierung der synthetischen DNA-Kassetten in den pKK233-2 Vektor.

Kloniertes  'AHB'-Gen (194 BP)

```
 -35                  -10                                    -SD-          Met Val Phe
GCTGTTGACAATTAATCATCCGGATCGTATAATGTGTGGAATTGTGAGCGGATAACAATTTCACACAGGAAACAGACCATGGTT TTC
                                                                              - Nco1 -
```

```
Trp Tyr Leu Val Ile Lys Val Ile Arg Leu Arg Leu Thr His Lys His Thr Leu Ile Thr Val Val Val
TGG TAC CTG GTT ATC AAA GTT ATC CGT CTG CGT CTG ACC CAC AAA CAC ACC CTG ATC ACC GTT GTT GTG
                                                                        - Bcl1 -
```

```
Val Leu Leu Val Ile Val Leu Ile Leu Ile Leu Ile Pro Gly Ser Ala Ala Ser Ala Asp Asp Ala Gly
GTT CTG CTG GTT ATC GTG CTG ATC CTG ATC CTG ATC CCG GGC TCC GCG GCT TCC GCA GAC GAC GCA GGT
                                                     - Xma1 -
```

```
Pro Pro Gly Ala Glu Ala Ala Glu Ala Ala Gly Ala Cys ***        rrn B (posn. 6416, ref. #66 )
CCG CCG GGT GCT GAG GCA GCT GAG GCT GCA GGT GCA TGC TGA AGCTTGGCTGTTTGGCGG
                                                  -Hind3-
```

Fig. 4  DNA-Sequenz der AHB-Kassette im Plasmid pKK-AHB.

```
        Met  Val  Phe  Trp  Tyr  Leu  14 Codons  Leu  Ile  Thr  STOP
5'— C C A T G G T T T T C T G G T A C C T G ————————— C T G A T C A C C T G A A G C T T —— 3'
3'— G G T A C C A A A A G A C C A T G G A C ————————— G A C T A G T G G A C T T C G A A —— 5'
    ——NcoI————              ———KpnI————            ———BclI————       ——HindIII——
```

Fig. 5.  Restriktionsstellen der DNA-A-Kassette in pKK-A.

```
              Ile  Leu  Arg  Met
      5'--- T G A T C C T G C G          3'
      3'--- A C T A G G A C G C G T A C_OH  5'
          --BclI*--         --NcoI*--
```

Fig. 6.  Umwandlung der BclI-Stelle durch zwei chemisch synthetisierte
         Nonamere (unterstrichen) als Adaptoren. Auf diese Weise wird
         die BclI-Stelle in eine BclI*-Stelle umgewandelt und ein
         kohäsives Ende für NcoI gebildet, das nach Ligation mit einem
         dazu passenden NcoI-Ende zu einer NcoI*-Stelle führt.

Fig. 7. Schema zur Verdeutlichung der Duplikationsstrategie der erfindungsgemäßen DNA-Kassetten.

BamHI
GGATCCGGAGCTTATCGACTGCACGGTGCACCAATGCTTCTGGCGTCAGGCAGCCATCGGAAGCTGTGGTATGGCT

GTGCAGGTCGTAAATCACTGCATAATTCGTGTCGCTCAAGGCGCACTCCCGTTCTGGATAATGTTTTTTGCGCCGA
             -35       -10 Ptac
CATCATAACGGTTCTGGCAAATATTCTGAAATGAGCTGTTGACAATTAATCATCGGCTCGTATAATGTGTGGAATT
 lac operator       Eco RI
GTGAGCGGATAACAATTTCACACAGGAAACAGAATTCTTGAAGACGAAAGGGCCTCGTGATACGCCTATTTTTATA
          Aat II
GGTTAATGTCATGATAATAATGGTTTCTTAGACGTCCGGTGGCACTTTTCGGGGAAATGTGCGCGGAACCCCTATT
      -35         -10 Pbla
TGTTTATTTTTCTAAATACATTCAAATATGTATCCGCTCATGAGACAATAACCCTGATAAATGCTTCAATAATATT
  SD    start     Nco I   HindIII
GAAAAAGGAAGAGTATGAGTATTCAAGCAGCCATGGCTGCCAAGCTTGGCTGTTTTGGCGGATCAGAGAATATTTT

CAGCCTGATACAGATTAAATCAGAACGCAGAAGCGGTCTGATAAAACAGAATTTGCCTGGCGGCAGTAGCGCGGTG
          5S rRNA
GTCCCACCTGACCCCATGCCGAACTCAGAAGTGAAACGCCGTAGCGCCGATGGTAGTGTGGGGTCTCCCCATGCGA
                   T1
GAGTAGGGAACTGCCAGGCATCAAATAAAACGAAAGGCTCAGTCGAAAGACTGGGCCTTTCGTTTTATCTGTTGTT

TGTCGGTGAACGCTCTCCTGAGTAGGACAAATCCGCCGGGAGCGGATTTGAACGTTGCGAAGCAACGGCCCGGAGG
                                   T2
GTGGCGGGCAGGACGCCCGCCATAAACTGCCAGGCATCAAATTAAGCAGAAGGCCATCCTGACGGATGGCCTTTTT

GCGTTTCTACAAACTCTT

Fig. 8 pGFY221N Restriktionskarte und DNA-Sequenz im Bereich der
      Klonierungsposition (NcoI/HindIII) des Vektors pGFY221N.

BamHI

GGATCCGGAG CTTATCGACT GCACGGTGCA CCAATGCTTC TGGCGTCAGG CAGCCATCGG AAGCTGTGGT

ATGGCTGTGC AGGTCGTAAA TCACTGCATA ATTCGTGTCG CTCAAGGCGC ACTCCCGTTC TGGATAATGT
                                                                    -35
TTTTTGCGCC GACATCATAA CGGTTCTGGC AAATATTCTG AAATGAGCTG TTGACAATTA ATCATCGGCT
    -10 Ptac                                                    EcoRI
CGTATAATGT GTGGAATTGT GAGCGGATAA CAATTTCACA CAGGAAACAG AATTCTAATT ACCAACACTA
                      SD  :      NcoI          HindIII
CTACGTTTTA ACTGAAACAA ACTGGAGACT GCCATGGCTG CCAAGCTTGG CTGTTTTGGC GGATCAGAGA

AGATTTTCAG CCTGATACAG ATTAAATCAG AACGCAGAAG CGGTCTGATA AAACAGAATT TGCCTGGCGG

CAGTAGCGCG GTGGTCCCAC CTGACCCCAT GCCGAACTCA GAAGTGAAAC GCCGTAGCGC CGATGGTAGT
                                                                    T1
GTGGGGTCTC CCCATGCGAG AGTAGGGAAC TGCCAGGCAT CAAATAAAAC GAAAGGCTCA GTCGAAAGAC

TGGGCCTTTC GTTTTATCTG TTGTTTGTCG GTGAACGCTC TCCTGAGTAG GACAAATCCG CCGGGAGCGG

ATTTGAACGT TGCGAAGCAA CGGCCCGGAG GGTGGCGGGC AGGACGCCCG CCATAAACTG CCAGGCATCA
          T2
AATTAAGCAG AAGGCCATCC TGACGGATGG CCTTTTTGCG TTTCTACAAA CTCTT

Fig. 9 pBIO52 Restriktionskarte und DNA-Sequenz im Bereich der Klonierungsposition (NcoI/HindIII) des Vektors pBIO52.

Fig. 10. Plasmid pUR300, modifizierter Vektor pUR292, nach Insertion einer neuen einzigen NcoI Restriktionsstelle. Die DNA-Sequenz an der Klonierungsstelle ist herausgeschrieben.

Fig. 11: Expression der für die artifiziellen Proteine codierenden Gene, kloniert in den Vektoren pGFY221N und pBIO52, in einem "in vitro" Transkriptions-Translation-System. Autoradiographie der Elektrophorese ($^{35}$S)-Methionin markierter Polypeptide (15 %iges Polyacrylamid-SDS-Gel).

Bahn 1: "In vitro" Transkriptions-Translation-System mit ($^{35}$S)-Met ergänzt, ohne DNA;

2: pBIO52; 3: pGFY221N; 4: pBIO52/A; 5: pGFY221N/A

6: pBIO52/AB; 7: pGFY221N/AB; 8: pBIO52/AF⁻B; 9: pGFY211N/AF⁻B;

10: pBIO52/AHB; 11: pGFY221N/AHB; M Molekulargewicht-Standards

Fig. 12: Beweis der Vollständigkeit der Aminosäuresequenzen artifizieller Proteine. Die Polypeptide wurden unter Verwendung der Vektoren pBIO52 und pGFY221N in Gegenwart von ($^{35}$S)-Methionin oder ($^{35}$S)-Cystein in einem "in vitro" Transkriptions-Translation-System exprimiert.

A : Bahn 1: pBIO52 ($^{35}$S) Met-markiert; Bahn 2: pBIO52 ($^{35}$S) Cys-markiert; Bahn 3: pBIO52/AB ($^{35}$S) Met-markiert; Bahn 4: pBIO52/AB ($^{35}$) Cys-markiert; Bahn 5: pBIO52/AF⁻B ($^{35}$) Met-markiert; Bahn 6: pBIO52/AF⁻B ($^{35}$S) Cys-markiert; Bahn 7: pBIO52/AHB ($^{35}$S) Met-markiert; Bahn 8: pBIO52/AHB ($^{35}$S) Cys-markiert.

B : Bahn 1: pGFY221N ($^{35}$S) Met-markiert; Bahn 2: pGFY221N/AB ($^{35}$S) Met-markiert; Bahn 3: pGFY221N/AB ($^{35}$S) Cys-markiert; Bahn 4: pGFY221N/AF⁻B($^{35}$S) Met-markiert; Bahn 5: pGFY221N/AF⁻B ($^{35}$S) Cys-markiert; M: ($^{14}$C)-markierte Molekulargewichtstandards. Autoradiographie der Elektrophorese auf dem 15 %igen SDS-Polyacrylamidgel.

Fig. 13: Vergleich der Translationsprodukte "in vitro" und "in vivo" von entsprechenden Genen unter Verwendung der Vektoren pBI052 (A) und pGFY221N (B). Die ungeraden Bahnen enthalten ($^{35}$S) Methionin-markierte Produkte der "in vitro" Transkriptions-Translation und die geraden Bahnen enthalten die Proteine vom Mini-Zellen-System nach der ($^{35}$S) Methionin-Markierung.

Bahnen 1 und 2: pGFY221N; Bahnen 3 und 4: pGFY221N/A; Bahnen 5 und 6: pGFY221N/AB; Bahnen 7 und 8: pGFY221N/AHB; Bahnen 9 und 10: pBI052; Bahnen 11 und 12: pBI052/A; Bahnen 13 und 14: pBI052/AB; Bahnen 15 und 16: pBI052/AF⁻B; Bahnen 17 und 18: pBI052/AHB. M:($^{14}$C)-markierte Molekulargewicht-Standards.

Autoradiographie der Elektrophorese ($^{35}$S)-Methionin-markierter Polypeptide (15 %iges Polyacrylamid-SDS-Gel).

Fig. 14: Protein-Expression im "in vitro" Transkriptions-Translation-System unter Verwendung der Vektoren pUR300, pUR300/AHB und pUR300/A6, Bahnen 1, 2 und 3. $^{35}$S-Methionin-markierte Proteine wurden auf dem 15 %igen SDS-Polyacrylamid-Gel analysiert.

Fig. 15: Expression der ß-Galactosidase (ß-Gal) und der Fusionsproteine ß-Gal-AHB und ß-Gal-A6 im E.coli Stamm BMH 71-18, unter Verwendung der Vektoren pUR300, pUR300/AHB und pUR300/A6.

Bahn 1: gereinigte ß-Galaktosidase (Sigma) als Molekulargewicht-Standard; Bahnen 2 und 3: pUR300 ohne und nach der IPTG Induktion; Bahnen 4 und 5; pUR300/AHB ohne und nach der IPTG Induktion; Bahnen 6 und 7: pUR300/A6 ohne und nach der IPTG Induktion.

Die Proben wurden auf dem 7,5 %igen SDS-Polyacrylamidgel analysiert.

## Europäisches Patentamt

# EUROPÄISCHER RECHERCHENBERICHT

Nummer der Anmeldung

EP 87 10 1633

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | TRENDS IN BIOTECHNOLOGY, Band 4, Nr. 12, Dezember 1986, Seiten 314-320, Elsevier Science Publishers, B.V., Amsterdam, NL; J.M. JAYNES et al.: "Plant protein improvement by genetic engineering: use of synthetic genes" <br> * Insgesamt; insbesondere, Figur 3 * | 1,54, 68,73 76 | C 12 N 15/00 <br> C 07 K 13/00 <br> C 12 P 21/02 <br> C 12 P 19/34 |
| | --- | | |
| X | FR-A-2 592 060 (NAUTILUS) <br><br> * Insgesamt * | 1,54, 68 | |
| | --- | | |
| X,D | GB-A-2 068 971 (G.D. SEARLE & CO.) <br> * Insgesamt * | 1,54, 68 | |

RECHERCHIERTE SACHGEBIETE (Int. Cl.4)

C 12 N
C 12 P

-----

Der vorliegende Recherchenbericht wurde für alle Patentansprüche erstellt.

| Recherchenort | Abschlußdatum der Recherche | Prüfer |
|---|---|---|
| DEN HAAG | 02-10-1987 | CUPIDO M. |

**Henkel, Feiler, Hänzel & Partner** — / — **Patentanwälte**

Dr. phil. G. Henkel
Dr. rer. nat. L. Feiler
Dipl.-Ing. W. Hänzel
Dipl.-Ing. D. Kottmann

Möhlstraße 37
D-8000 München 80

Tel.: 089/982085-87
Telex: 529802 hnkl d
Telefax (Gr. 2+3):
089/981426
Telegramm: ellipsoid

EUROPÄISCHES PATENTAMT

8000 München 2

3. April 1987

87 101 633.3
BIOSYNTECH Biochemische Synthesetechnik GmbH
Unser Zeichen: Dr.F/pi

~~Auf die Mitteilung gemäß Teil A, Kapitel IV, 4.2 der~~
~~Richtlinien für die Prüfung im Europäischen Patentamt:~~

Anliegend werden neue Beschreibungsseiten 20 und 23 ~~sowie~~
~~neue Anspruchsseiten 5 und 6~~ mit der Bitte überreicht,
die entsprechenden ursprünglichen Beschreibungs- ~~und~~
~~Anspruchs~~seiten durch die neu eingereichten Beschreibungs-
~~und Anspruchssei~~ten zu ersetzen. ~~Auf der neuen~~
~~Beschreibungsseite 23 sowie den neuen Anspruchsseiten 5~~
~~(Anspruch 44) und 6 (Ansprüche 49, 50 und 53) wurden die~~
~~Angaben nach Regel 28(1)(c) EPÜ gemacht.~~ Die Richtigstellung der Bezeichnung des Wirts der bei der
Deutschen Sammlung von Mikroorganismen hinterlegten Wirt-
Vektor-Systeme von CMK 603 in DS 410 auf der ursprünglichen Beschreibungsseite 23 dürfte nach diesseitiger
Auffassung keinen Verstoß gegen die Vorschrift des
Artikel 123(2) EPÜ darstellen, da es für die Erfüllung
von Regel 28(1)(a) EPÜ auf die tatsächliche Hinterlegung
spätestens am Anmeldetag ankommt und die Angaben über
die Hinterlegungsstelle und das Aktenzeichen der
Hinterlegung gemäß Regel 28(2) EPÜ nachgereicht werden
können. In Übereinstimmung mit der vorgenommenen

Richtigstellung war dann auch auf Seite 20 in der drittletzten Zeile "CMK 603" durch "DS 410" zu ersetzten.

~~Beigefügt sind ferner die mit der genannten Mitteilung angeforderten Bescheinigungen über die Hinterlegung der auf der beigefügten Beschreibungsseite 23 genannten Wirt-Vektor-Systeme bei der Deutschen Sammlung von Mikroorganismen, Grisebachstraße 8, Göttingen.~~

Patentanwalt

Anlagen
wie erwähnt